# EUROPEAN PATENT APPLICATION

(11) **EP 4 806 881 A1**
(43) Date of publication of application: **16.09.2026**
(21) Application number: 24889016.2
(22) Date of filing: 29.10.2024
(51) Int. Cl.: C07C 219/06, C07C 229/16, C07D 213/73, C07C 323/12, A61K 47/14, A61K 47/18, A61K 47/22, A61K 9/51, A61K 39/00, A61K 39/085

(54) **NOVEL IONIZABLE LIPID AND LIPID NANOPARTICLE COMPOSITION USING SAME**

(30) Priority: 06.11.2023 KR 20230151386; 16.05.2024 KR 20240063939
(71) Applicant: Medicibio Co., Ltd, Yongin-si, Gyeonggi-do 17095 (KR)
(72) Inventor: KI, Min Hyo, Yongin-si Gyeonggi-do 17095 (KR); CHOI, Mee-Hwa, Yongin-si Gyeonggi-do 16874 (KR); PARK, So Hyun, Yongin-si Gyeonggi-do 17011 (KR); LEE, Kug Hwa, Yongin-si Gyeonggi-do 17084 (KR)
(74) Representative: Roos, Peter
(86) International application number: PCT/KR2024/016684
(87) International publication number: WO 2025/100828

(57) **Abstract**

The present invention relates to novel ionizable lipids and lipid nanoparticle compositions comprising the same. Lipid nanoparticles comprising the novel ionizable lipids exhibit excellent encapsulation efficiency and high intracellular and *in vivo* gene delivery efficiency, and also exhibit excellent muscle delivery upon local administration, thereby being useful as compositions for drug delivery.

## Description

### Technical Field

The present invention relates to novel ionizable lipids and lipid nanoparticle compositions comprising the same.

### Background Art

Nucleic-acid-based drugs are used not only as therapeutic agents but also as prophylactic agents for preventing disease by administering genes capable of expressing antigens against a particular disease. Gene-based vaccines are generally classified as DNA vaccines, RNA vaccines, and viral-vector vaccines. Among these, RNA vaccines involve administration of mRNA encoding an antigen so that the antigen is expressed *in vivo*, thereby inducing antibody formation. RNA vaccines have attracted considerable attention as an effective response to COVID-19 because they can be rapidly developed and avoid the potential risks associated with viral-vector vaccines and the possibility of genomic mutation associated with DNA vaccines.

However, genes are macromolecules that are readily degraded by nucleases *in vivo* and, due to their negatively charged macromolecules, are not easily delivered into cells. Accordingly, there remains a need for methods capable of stably and efficiently delivering nucleic acids to a desired site. Various delivery systems based on lipids, polymers, dendrimers, inorganic metal materials, and the like have been reported. Notably, patisiran, the first siRNA drug approved by the FDA in 2018, and the mRNA vaccines for COVID-19 approved for emergency use in 2020 both employ lipid nanoparticles. Conventional lipid nanoparticles generally comprise four components mixed in defined ratios: an ionizable lipid, a phospholipid (helper lipid), cholesterol (a structural lipid), and a PEG-lipid.

As lipid-nanoparticle-based delivery systems have been commercialized through siRNA therapeutics and mRNA vaccines, new challenges have emerged, including improved stability and cellular uptake, targeting capability, ease of storage and distribution, mitigation of adverse effects, cost reduction, and response to breakthrough infections. There is therefore a continuing need in the nucleic-acid therapeutics field for novel lipid nanoparticles capable of addressing these issues.

Recent studies and extensive clinical experience with mRNA vaccines have established that the efficacy of lipid-nanoparticle-based mRNA vaccines is achieved through local action at the administration site. After intramuscular administration of lipid nanoparticles comprising antigen-encoding mRNA, the immune response is established through: (1) antigen expression in nearby muscle cells; (2) antigen expression in antigen-presenting cells, such as dendritic cells, macrophages, and Langerhans cells, present around muscle tissue; and (3) activation of B cells and T cells in lymph nodes surrounding the muscle tissue.

At the same time, it has been reported that, in developing lipid-nanoparticle-based mRNA vaccines, it is important to balance immunogenicity as the desired therapeutic effect with systemic reactogenicity as an undesirable adverse effect. In particular, excessive systemic reactogenicity associated with mRNA vaccines are believed to be related to systemic circulation of lipid nanoparticles.

Accordingly, although lipid-nanoparticle-based mRNA vaccines exert their immune effects primarily through local mechanisms, unnecessary systemic exposure of locally administered lipid nanoparticles may cause various side effects. For this reason, muscle-targeting lipid nanoparticle technology is required for the development of optimal mRNA vaccines. Nevertheless, lipid nanoparticle technology capable of minimizing systemic exposure while providing locally concentrated delivery has not yet been fully established and remains an unmet need in the development of mRNA vaccines for infectious disease and cancer.

### Disclosure of the Invention

### Technical Goals

An object of the present invention is to provide a novel compound, or a pharmaceutically acceptable salt, tautomer, prodrug, or stereoisomer thereof.

Another object of the present invention is to provide a lipid nanoparticle composition comprising the compound.

Still another object of the present invention is to provide a drug-delivery composition comprising the lipid nanoparticle composition and a prophylactic or therapeutic agent.

### Technical Solutions

In order to achieve the foregoing objects, the present invention provides a compound represented by Chemical Formula 1, or a pharmaceutically acceptable salt, tautomer, or stereoisomer thereof. 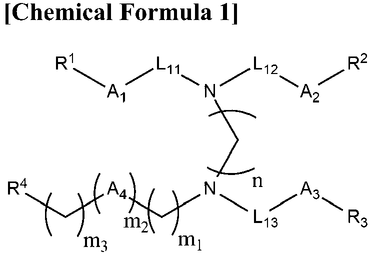 In Chemical Formula 1,
n is one selected from 1 to 6;
m₁ and m₃ are each independently one selected from 0 to 6;
m₂ is 0 or 1;
L₁₁, L₁₂, and L₁₃ are each the same or different, and selected from the group consisting of (C1-C10)alkylene, (C2-C10)alkenylene, and (C2-C10)alkynylene, wherein at least one -CH₂-groups in the alkylene, alkenylene, and alkynylene are optionally substituted with -O-, -S-, or - SS-;
A₁ to A₄ are each the same or different, and selected from the group consisting of -C(=O)-, - OC(=O)-, -C(=O)O-, -OC(=O)O-, -OP(=O)O-, -OC(=S)-, -C(=S)O-, -SC(=O)-, -C(=O)S-, - SC(=S)-, -C(=S)S-, -SC(=O)O-, -OC(=O)S-, -S-, -SS-, -C(=O)NH-, -NHC(=O)-, -C(=O)NR¹¹-, -NR¹¹C(=O)-, -OC(=O)NH-, -NHC(=O)O-, -OC(=O)NR¹¹-, and -NR¹¹C(=O)O-; R¹¹ is selected from the group consisting of (C1-C10)alkyl, (C2-C10)alkenyl, and (C2-C10)alkynyl, wherein at least one -CH₂- groups in the alkyl, alkenyl, and alkynyl are optionally substituted with -O-, -S-, or -SS-; and
R¹, R², R³, and R⁴ are each the same or different, and selected from the group consisting of hydroxy, (C1-C20)alkyl, (C2-C20)alkenyl, (C2-C20)alkynyl, (C3-C20)cycloalkyl, (C3-C20)aryl, and 4- to 20-membered heteroaryl comprising at least one heteroatom selected from the group consisting of N, O, and S, wherein at least one -CH₂- groups in the alkylene, alkenylene and alkynylene is optionally substituted with -O-, -S- or -SS-, and wherein the (C3-C20)cycloalkyl, (C3-C20)aryl, and 4- to 20-membered heteroaryl groups are each optionally substituted with at least one substituent selected from hydroxy, amino, halogen, and (C1-C5)alkyl.

In another aspect, the present invention provides a lipid nanoparticle composition comprising the compound represented by Chemical Formula 1, or a pharmaceutically acceptable salt, tautomer, or stereoisomer thereof.

In still another aspect, the present invention provides a drug-delivery composition comprising the lipid nanoparticle composition and a prophylactic or therapeutic agent.

### Advantageous Effects

According to the present invention, lipid nanoparticles comprising the novel ionizable lipids exhibit excellent encapsulation efficiency and high *in vitro* and *in vivo* gene delivery efficiency. In addition, upon local administration, the lipid nanoparticles show excellent muscle delivery while minimizing systemic exposure, thereby making them useful as drug-delivery compositions.

### Brief Description of Drawings

FIG. 1 shows an exemplary synthetic scheme of preparing Compound 1 (Example 1-1) of the present invention.
FIG. 2 shows an exemplary synthetic scheme of preparing compound 2 (Example 1-2) of the present invention.
FIG. 3 shows an exemplary synthetic scheme of preparing compound 3 (Example 1-3) of the present invention.
FIG. 4 shows an exemplary synthetic scheme of preparing compound 4 (Example 1-4) of the present invention.
FIG. 5 shows an exemplary synthetic scheme of preparing compound 5 (Example 1-5) of the present invention.
FIG. 6 shows an exemplary synthetic scheme of preparing compound 6 (Example 1-6) of the present invention.
FIG. 7 shows an exemplary synthetic scheme of preparing compound 7 (Example 1-7) of the present invention.
FIG. 8 shows an exemplary synthetic scheme of preparing compound 8 (Example 1-8) of the present invention.
FIG. 9 shows an exemplary synthetic scheme of preparing compound 9 (Example 1-9) of the present invention.
FIG. 10 shows an exemplary synthetic scheme of preparing compound 10 (Example 1-10) of the present invention.
FIG. 11 shows an exemplary synthetic scheme of preparing compound 11 (Example 1-11) of the present invention.
FIG. 12 shows an exemplary synthetic scheme of preparing compound 12 (Example 1-12) of the present invention.
FIG. 13 shows an exemplary synthetic scheme of preparing compound 13 (Example 1-13) of the present invention.

### Best Mode for Carrying Out the Invention

The present invention is described in further detail below.

The present invention provides a compound represented by the following Chemical Formula 1, a pharmaceutically acceptable salt thereof, a tautomer or a stereoisomer thereof. 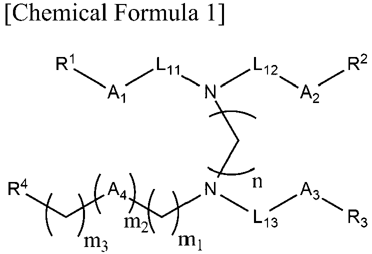 In Chemical Formula 1,
n is an integer selected from 1 to 6;
m₁ and m₃ are each independently an integer selected from 0 to 6;
m₂ is 0 or 1;
L₁₁, L₁₂, and L₁₃ are each the same or different, and selected from the group consisting of (C1-C10)alkylene, (C2-C10)alkenylene, and (C2-C10)alkynylene, wherein at least one -CH₂-groups in the alkylene, alkenylene, and alkynylene are optionally substituted with -O-, -S-, or - SS-;
A₁ to A₄ are each the same or different, and selected from the group consisting of -C(=O)-, - OC(=O)-, -C(=O)O-, -OC(=O)O-, -OP(=O)O-, -OC(=S)-, -C(=S)O-, -SC(=O)-, -C(=O)S-, - SC(=S)-, -C(=S)S-, -SC(=O)O-, -OC(=O)S-, -S-, -SS-, -C(=O)NH-, -NHC(=O)-, -C(=O)NR¹¹-, -NR¹¹C(=O)-, -OC(=O)NH-, -NHC(=O)O-, -OC(=O)NR¹¹-, and -NR¹¹C(=O)O-; R¹¹ is selected from the group consisting of (C1-C10)alkyl, (C2-C10)alkenyl, and (C2-C10)alkynyl, wherein at least one -CH₂- groups in the alkyl, alkenyl, and alkynyl are optionally substituted with -O-, -S-, or -SS-; and
R¹, R², R³, and R⁴ are each the same or different, and selected from the group consisting of hydroxy, (C1-C20)alkyl, (C2-C20)alkenyl, (C2-C20)alkynyl, (C3-C20)cycloalkyl, (C3-C20)aryl, and 4- to 20-membered heteroaryl comprising at least one heteroatom selected from the group consisting of N, O, and S, wherein at least one -CH₂- groups in the alkylene, alkenylene and alkynylene is optionally substituted with -O-, -S- or -SS-, and wherein the (C3-C20)cycloalkyl, (C3-C20)aryl, and 4- to 20-membered heteroaryl groups are each optionally substituted with at least one substituent selected from hydroxy, amino, halogen, and (C1-C5)alkyl.

In Chemical Formula 1, n is selected from 2 to 4; m₁ and m₃ are each independently selected from 0 to 4; L₁₁, L₁₂, and L₁₃ are each the same or different, and selected from the group consisting of (C3-C6)alkylene, (C3-C6)alkenylene, and (C3-C6)alkynylene; A₁ to A₄ are each the same or different, and selected from -OC(=O)- or -C(=O)O-; R¹, R², and R³ are each the same or different, and selected from the group consisting of (C5-C20)alkyl, (C5-C20)alkenyl, and (C5-C20)alkynyl, wherein at least one -CH₂- groups are optionally substituted with -O-, - S-, or -SS-; and R⁴ is hydroxy, (C3-C10)cycloalkyl, (C3-C10)aryl, or a 4- to 10-membered heteroaryl comprising at least one nitrogen atom, wherein the (C3-C10)cycloalkyl, (C3-C10)aryl, and 4- to 10-membered heteroaryl groups are each optionally substituted with amino.

Preferably, in Chemical Formula 1, n is selected from 3 to 4; L₁₁, L₁₂, and L₁₃ are each independently (C3-C6)alkylene; R¹, R², and R³ are each the same or different, and selected from the group consisting of (C5-C20)alkyl, (C5-C20)alkenyl, and (C5-C20)alkynyl, wherein at least one -CH₂- groups are optionally substituted with -S- or -SS-; and R⁴ is hydroxy or a 4-to 10-membered heteroaryl comprising one nitrogen atom, wherein the heteroaryl group is optionally substituted with amino.

More preferably, the compound may be selected from the following group of compounds.
(1) (9Z,9'Z)-((3-((2-hydroxyethyl)(3-((Z)-tetradec-9-enoyloxy)propyl)amino) propyl)azanediyl)bis(propane-3,1-diyl)bis(tetradec-9-enoate);
(2) (9Z,9'Z)-((3-((3-((Z)-hexadec-9-enoyloxy)propyl)(2-hydroxyethyl)amino)propyl)azanediyl)bis(propane-3,1-diyl) bis(hexadec-9-enoate);
(3) di((Z)-dec-4-en-1-yl)4,4'-((3-((4-((Z)-dec-4-en-1-yloxy)-4-oxobutyl)(2-hydroxyethyl)amino)propyl)azanediyl)dibutanoate;
(4) didodecyl 4,4'-((3-((4-(dodecyloxy)-4-oxobutyl) (2-hydroxyethyl)amino)propyl)azanediyl)dibutanoate;
(5) di((Z)-dec-4-en-1-yl)4,4'-((3- ((2-((3-(6-aminopyridin-3-yl) propanoyl)oxy)ethyl)(4-((Z)-dec-4-en-1-yloxy)-4-oxobutyl)amino)propyl)azanediyl)dibutanoate;
(6) di((Z)-dec-4-en-1-yl)4,4'-((4-((4-((Z)-dec-4-en-1-yloxy)-4-oxobutyl) (2-hydroxyethyl)amino)butyl)azanediyl)dibutanoate;
(7) di((Z)-dec-4-en-1-yl)5,5'-((3-((5-((Z)-dec-4-en-1-yloxy)-5-oxopentyl) (2-hydroxyethyl)amino)propyl)azanediyl)dipentanoate;
(8) dinonyl6,6'-((4-((2-hydroxyethyl)(6-(nonyloxy) -6-oxohexyl)amino)butyl)azanediyl)dihexanoate;
(9) heptadecan-9-yl 6-((4-((2- hydroxyethyl)(6-(nonyloxy)-6-oxohexyl)amino)butyl)(6-(nonyloxy)-6-oxohexyl)amino)hexanoate;
(10) didecyl 6,6'-((2-((6-(decyloxy)-6-oxohexyl) (2-hydroxyethyl)amino)ethyl)azanediyl)dihexanoate;
(11) decyl 6-((2-((6-(decyloxy) -6-oxohexyl)(2-hydroxyethyl)amino)ethyl)(6-((2-hexyldecyl)oxy)-6-oxohexyl)amino)hexanoate;
(12) bis(2-hexyldecyl) 6,6'-((2-((6-(decyloxy) -6-oxohexyl)(2-hydroxyethyl)amino)ethyl)azanediyl)dihexanoate;
(13) di((Z)-dec-4-en-1-yl) 4,4'-((3-((4-((Z)-dec-4-en-1-yloxy)-4-oxobutyl)(pyridin-4-yl)amino)propyl)azanediyl)dibutanoate;
(14) di((Z)-tetradec-9-en-1-yl) 4,4'-((3-((2-hydroxyethyl)(4-oxo-4-((Z)-tetradec-9-en-1-yloxy)butyl)amino)propyl)azanediyl)dibutanoate;
(15) bis(6-(butyldisulfanyl)hexyl) 4,4'-((3-((4-((6-(butyldisulfanyl)hexyl)oxy)-4-oxobutyl)(3-hydroxypropyl)amino)propyl)azanediyl)dibutanoate;
(16) di((Z)-dec-4-en-1-yl)4,4'-((3-((2-((3-(4-aminophenyl)propanoyl)oxy) ethyl)(4-((Z)-dec-4-en-1-yloxy)-4-oxobutyl)amino)propyl)azanediyl)dibutanoate;
(17) di((Z)-dec-4-en-1-yl) 4,4'-((3-((4-aminophenethyl)(4-((Z)-dec-4-en-1-yloxy)-4-oxobutyl)amino)propyl)azanediyl)dibutanoate;
(18) di((Z)-dec-4-en-1-yl) 4,4'-((3-((4-((Z)-dec-4-en-1-yloxy)-4-oxobutyl)(4-hydroxybutyl)amino)propyl)azanediyl)dibutanoate; and
(19) (9Z,9'Z)-((3-((2-hydroxyethyl)(4-((Z)-tetradec-9-enoyloxy)butyl)amino)propyl)azanediyl)bis(butane-4,1-diyl) bis(tetradec-9-enoate).

Compound 1: (9Z,9'Z)-((3-((2-hydroxyethyl)(3-((Z)-tetradec-9-enoyloxy)propyl)amino) propyl)azanediyl)bis(propane-3,1-diyl)bis(tetradec-9-enoate); Compound 1 has the structure of Chemical Formula 2 below, its molecular formula is C₅₆H₁₀₄N₂O₇, and its molecular weight is 917.43.

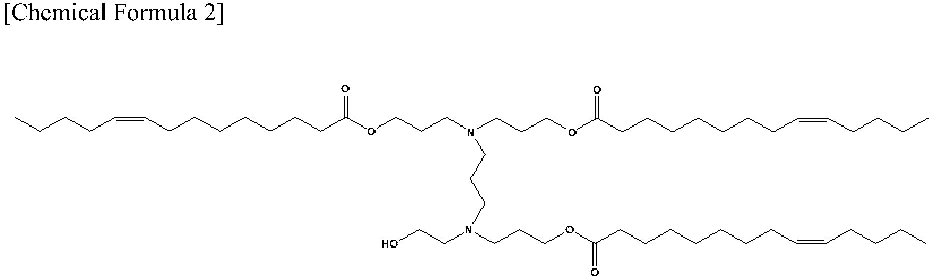

Compound 2: (9Z,9'Z)-((3-((3-((Z)-hexadec-9-enoyloxy)propyl)(2-hydroxyethyl)amino)propyl)azanediyl)bis(propane-3,1-diyl) bis(hexadec-9-enoate); Compound 2 has the structure of Chemical Formula 3 below, its molecular formula is C₆₂H₁₁₆N₂O₇, and its molecular weight is 1001.59.

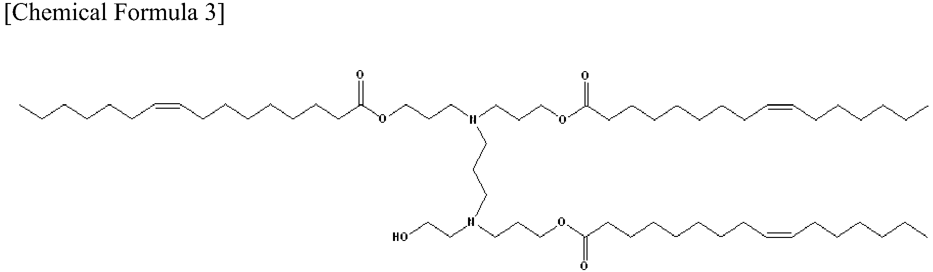

Compound 3: di((Z)-dec-4-en-1-yl)4,4'-((3-((4-((Z)-dec-4-en-1-yloxy)-4-oxobutyl)(2-hydroxyethyl)amino)propyl)azanediyl)dibutanoate; Compound 3 has the structure of Chemical Formula 4 below, its molecular formula is C₄₇H₈₆N₂O₇, and its molecular weight is 791.19.

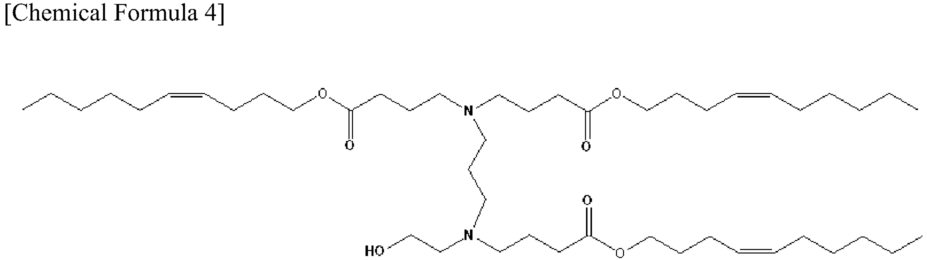

Compound 4: didodecyl 4,4'-((3-((4-(dodecyloxy)-4-oxobutyl) (2-hydroxyethyl)amino)propyl)azanediyl)dibutanoate; Compound 4 has the structure of Chemical Formula 5 below, its molecular formula is C₅₃H₁₀₄N₂O₇, and its molecular weight is 881.40.

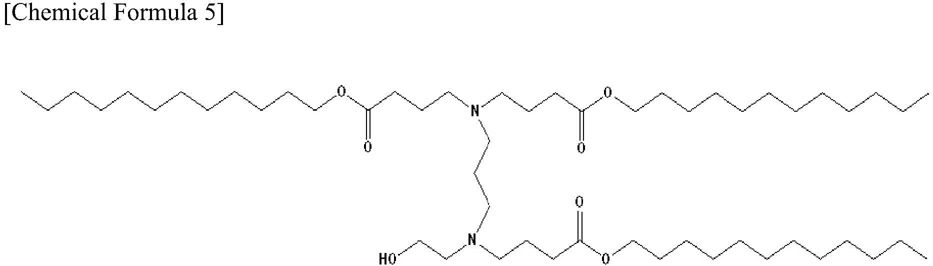

Compound 5: di((Z)-dec-4-en-1-yl)4,4'-((3- ((2-((3-(6-aminopyridin-3-yl) propanoyl)oxy)ethyl)(4-((Z)-dec-4-en-1-yloxy)-4-oxobutyl)amino)propyl)azanediyl)dibutanoate; Compound 5 has the structure of Chemical Formula 6 below, its molecular formula is C₅₅H₉₄N₄O₈, and its molecular weight is 939.36.

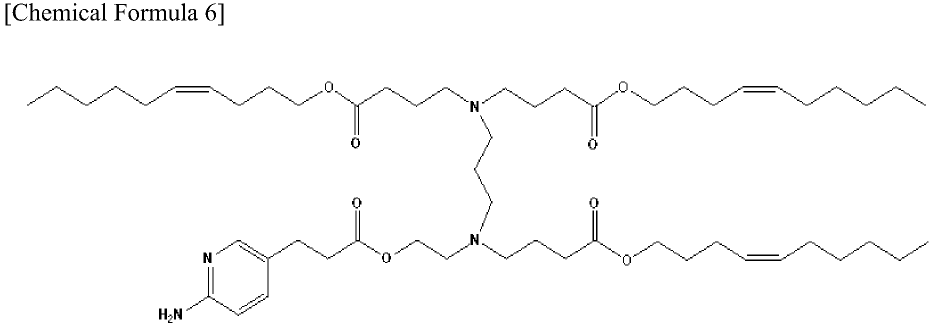

Compound 6: di((Z)-dec-4-en-1-yl)4,4'-((4-((4-((Z)-dec-4-en-1-yloxy)-4-oxobutyl) (2-hydroxyethyl)amino)butyl)azanediyl)dibutanoate; Compound 6 has the structure of Chemical Formula 7 below, its molecular formula is C₄₈H₈₈N₂O₇, and its molecular weight is 805.22.

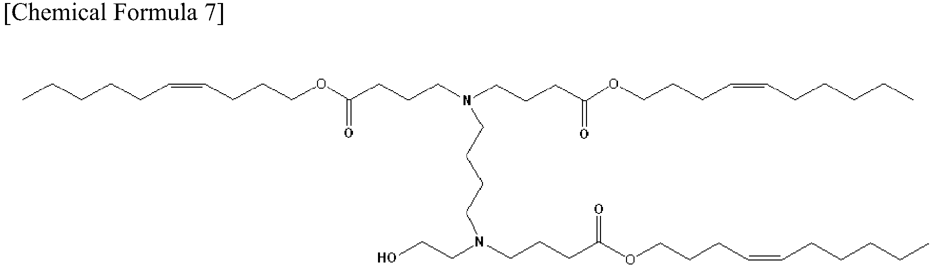

Compound 7: di((Z)-dec-4-en-1-yl)5,5'-((3-((5-((Z)-dec-4-en-1-yloxy)-5-oxopentyl) (2-hydroxyethyl)amino)propyl)azanediyl)dipentanoate; Compound 7 has the structure of Chemical Formula 8 below, its molecular formula is C₅₀H₉₂N₂O₇, and its molecular weight is 833.27.

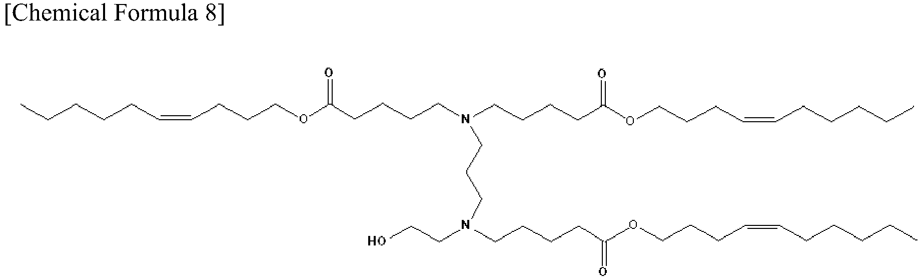

Compound 8: dinonyl6,6'-((4-((2-hydroxyethyl)(6-(nonyloxy) -6-oxohexyl)amino)butyl)azanediyl)dihexanoate; Compound 8 has the structure of Chemical Formula 9 below, its molecular formula is C₅₁H₁₀₀N₂O₇, and its molecular weight is 853.35.

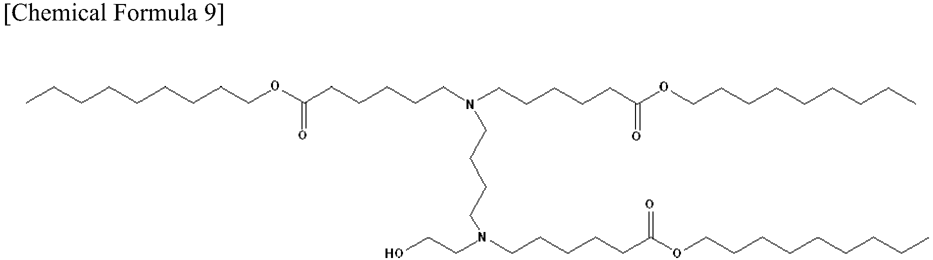

Compound 9: heptadecan-9-yl 6-((4-((2- hydroxyethyl)(6-(nonyloxy)-6-oxohexyl)amino)butyl)(6-(nonyloxy)-6-oxohexyl)amino)hexanoate; Compound 9 has the structure of Chemical Formula 10 below, its molecular formula is C₅₉H₁₁₆N₂O₇, and its molecular weight is 965.56.

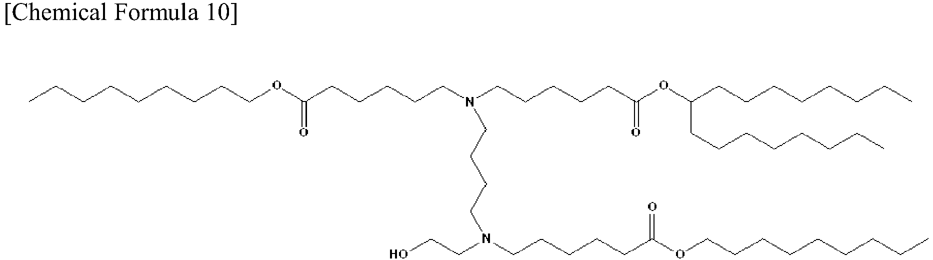

Compound 10: didecyl 6,6'-((2-((6-(decyloxy)-6-oxohexyl) (2-hydroxyethyl)amino)ethyl)azanediyl)dihexanoate; Compound 10 has the structure of Chemical Formula 11 below, its molecular formula is C₅₂H₁₀₂N₂O₇, and its molecular weight is 867.38.

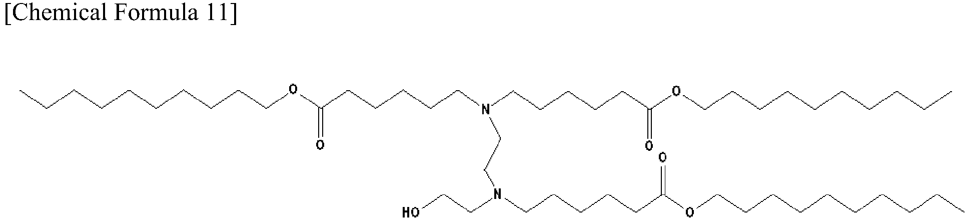

Compound 11: decyl 6-((2-((6-(decyloxy) -6-oxohexyl)(2-hydroxyethyl)amino)ethyl)(6-((2-hexyldecyl)oxy)-6-oxohexyl)amino)hexanoate; Compound 11 has the structure of Chemical Formula 12 below, its molecular formula is C₅₈H₁₁₄N₂O₇, and its molecular weight is 951.53.

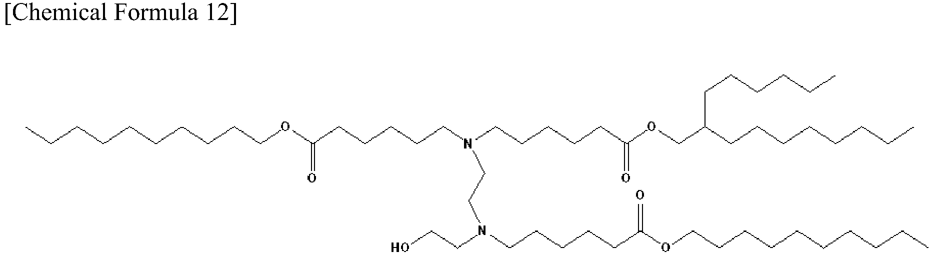

Compound 12: bis(2-hexyldecyl) 6,6'-((2-((6-(decyloxy) -6-oxohexyl)(2-hydroxyethyl)amino)ethyl)azanediyl)dihexanoate; Compound 12 has the structure of Chemical Formula 13 below, its molecular formula is C₆₄H₁₂₆N₂O₇, and its molecular weight is 1035.69.

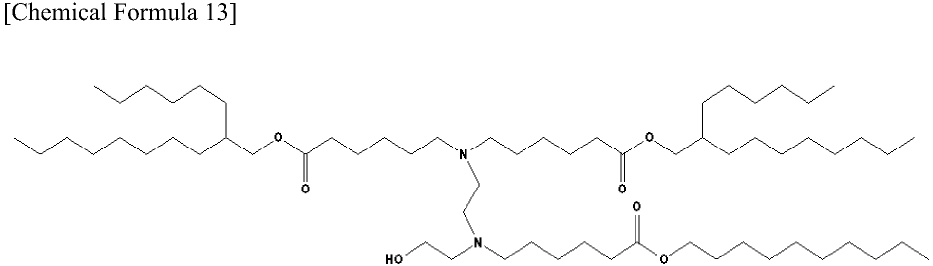

Compound 13: di((Z)-dec-4-en-1-yl) 4,4'-((3-((4-((Z)-dec-4-en-1-yloxy)-4-oxobutyl)(pyridin-4-yl)amino)propyl)azanediyl)dibutanoate; Compound 13 has the structure of Chemical Formula 14 below, its molecular formula is C₅₀H₈₅N₃O₆, and its molecular weight is 824.23.

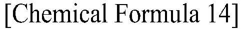

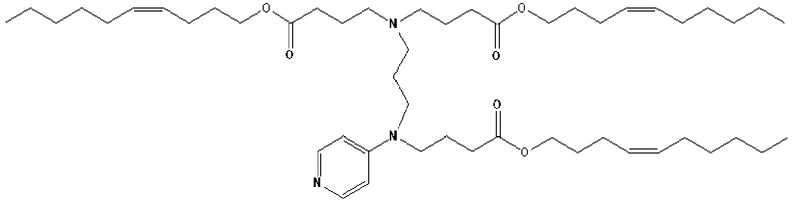

Compound 14: di((Z)-tetradec-9-en-1-yl) 4,4'-((3-((2-hydroxyethyl)(4-oxo-4-((Z)-tetradec-9-en-1-yloxy)butyl)amino)propyl)azanediyl)dibutanoate; Compound 14 has the structure of Chemical Formula 15 below, its molecular formula is C₅₉H₁₁₀N₂O₇, and its molecular weight is 959.51.

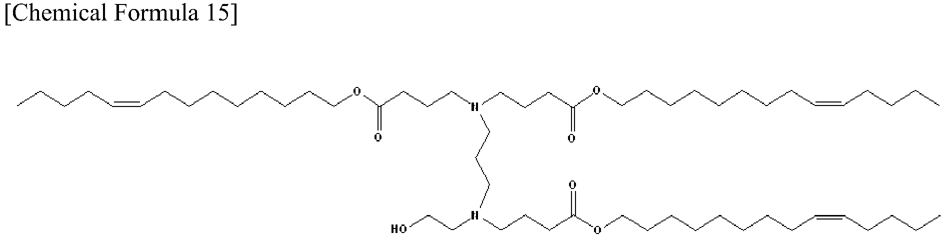

Compound 15: bis(6-(butyldisulfanyl)hexyl) 4,4'-((3-((4-((6-(butyldisulfanyl)hexyl)oxy)-4-oxobutyl)(3-hydroxypropyl)amino)propyl)azanediyl)dibutanoate; Compound 15 has the structure of Chemical Formula 16 below, its molecular formula is C₄₈H₉₄N₂O₇S₆, and its molecular weight is 1003.66.

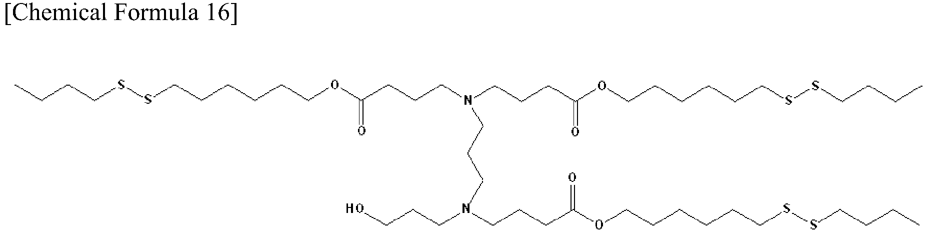

Compound 16: di((Z)-dec-4-en-1-yl)4,4'-((3-((2-((3-(4-aminophenyl)propanoyl)oxy) ethyl)(4-((Z)-dec-4-en-1-yloxy)-4-oxobutyl)amino)propyl)azanediyl)dibutanoate; Compound 16 has the structure of Chemical Formula 17 below, its molecular formula is C₅₆H₉₅N₃O₈, and its molecular weight is 938.37.

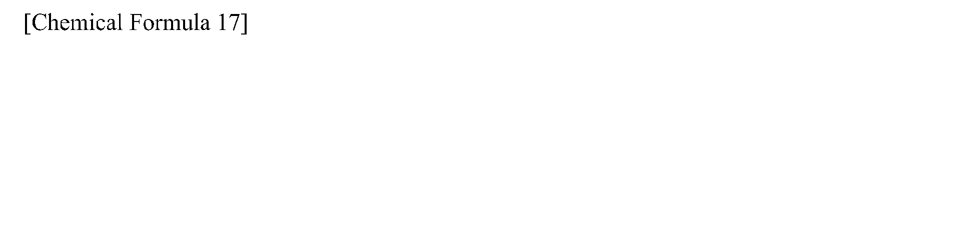

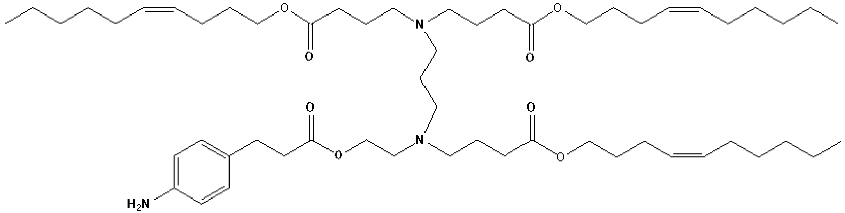

Compound 17: di((Z)-dec-4-en-1-yl) 4,4'-((3-((4-aminophenethyl)(4-((Z)-dec-4-en-1-yloxy)-4-oxobutyl)amino)propyl)azanediyl)dibutanoate; Compound 17 has the structure of Chemical Formula 18 below, its molecular formula is C₅₃H₉₁N₃O₆, and its molecular weight is 866.31.

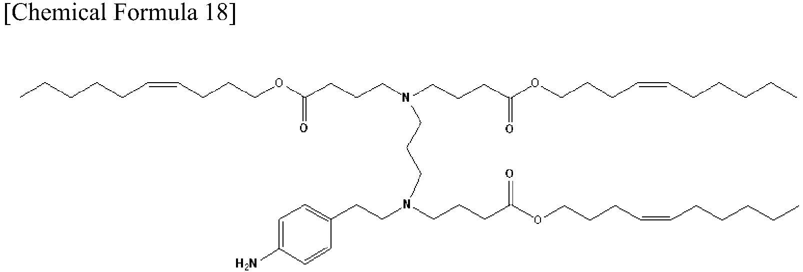

Compound 18: di((Z)-dec-4-en-1-yl) 4,4'-((3-((4-((Z)-dec-4-en-1-yloxy)-4-oxobutyl)(4-hydroxybutyl)amino)propyl)azanediyl)dibutanoate; Compound 18 has the structure of Chemical Formula 19 below, its molecular formula is C₄₉H₉₀N₂O₇, and its molecular weight is 819.25.

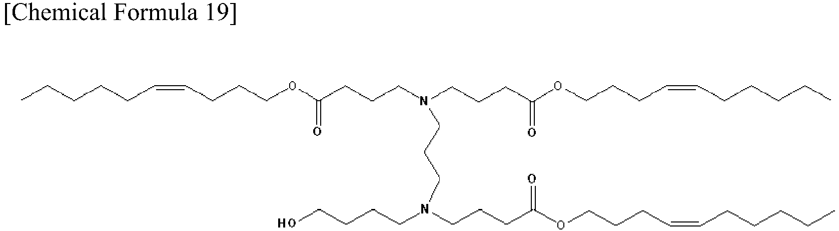

Compound 19: (9Z,9'Z)-((3-((2-hydroxyethyl)(4-((Z)-tetradec-9-enoyloxy)butyl)amino)propyl)azanediyl)bis(butane-4,1-diyl) bis(tetradec-9-enoate); Compound 19 has the structure of Chemical Formula 20 below, its molecular formula is C₅₉H₁₁₀N₂O₇, and its molecular weight is 959.51.

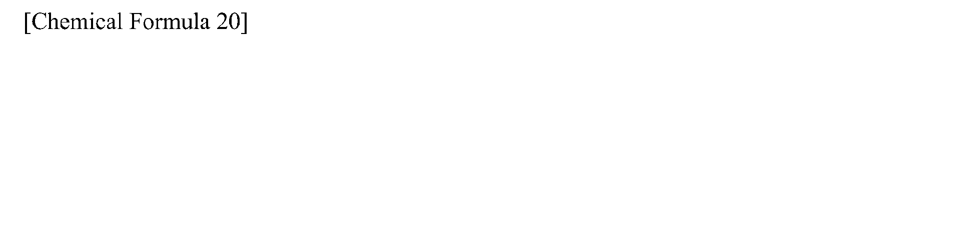

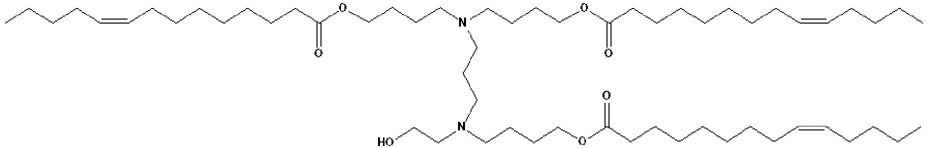

The present invention further provides a lipid nanoparticle composition comprising the compound represented by Chemical Formula 1, or a pharmaceutically acceptable salt, tautomer, or stereoisomer thereof.

The lipid nanoparticle has excellent safety and stability, and may function as a carrier so that a gene including RNA, DNA, or a mixed type thereof as an active ingredient shows its effect well in a cell. Accordingly, the lipid nanoparticles by the ionizable lipid exhibit excellent encapsulation efficiency and gene delivery efficiency in cells/*in vivo*, and on the other hand, exhibit excellent muscle delivery of genes upon local administration, and thus can be usefully used as a composition for drug delivery.

The compound may be an ionizable lipid, and the ionizable lipid may include a gene, a protein, or a compound, preferably a gene or a protein, and more preferably a gene.

The composition may further include one or more selected from the group consisting of neutral lipids, steroids, and polymerized lipids, but is not limited thereto.

The neutral lipid may be phospholipid or glycolipid. Specifically, the phospholipid may be at least one selected from the group consisting of dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), and dipalmitoylphosphatidylethanolamine The glycolipids may be at least one selected from the group consisting of glucosylceramide, galactosylceramide, glucosylsphingosine, galactosylsphingosine, and phosphoglycoceramide, but are not limited thereto.

The steroids may be at least one selected from the group consisting of cholesterol, bile acid derivatives, and cholic acid derivatives, but are not limited thereto.

The polymerizable lipid may be a PEGylated lipid having a structure in which a water-soluble polymer and a lipid are bonded, and the PEGylated lipid may be at least one selected from the group consisting of PEG conjugated to dialkyloxypropyl (PEG-DAA); PEG-c-DOMG and PEG-DMG as PEG conjugated to diacylglycerol (PEG-DAG); PEG-DLPE, PEG-DMPE, and PEG-DSPE as PEG conjugated to a phospholipid such as phosphatidylethanolamine (PEG-PE); PEG conjugated to ceramide (PEG-CER); PEG conjugated to cholesterol or a derivative thereof; PEG-modified phosphatidic acid; PEG-modified dialkylamine; and PEG-modified dialkylglycerol, but is not limited thereto. In addition, the PEGylated lipid may be a functionalized PEG in which a functional group is bonded to a side not bonded to a lipid. The functional group may be at least one selected from the group consisting of a succinyl group, a carboxylic acid, a maleimide, an n-hydroxysuccinimide, an amine group, a biotin, a cyanur group, and a folate, but is not limited thereto.

The ionizable lipid may be prepared as lipid nanoparticles including neutral lipid, steroids, polymerized lipids, and the like. For a composition suitable for preparing lipid nanoparticles, the lipid composition excluding the active ingredient may include 20 to 65 mol % of the ionizable lipid, 2.5 to 30 mol % of the neutral lipid, 20 to 60 mol % of the steroid, and 0.5 to 5 mol % of the polymerized lipid. For example, the ionizable lipid : neutral lipid : steroid : polymerized lipid may be formulated at a mol% ratio selected from the group consisting of 60:5:33.5:1.5, 50:10:38.5:1.5, 40:15:43.5:1.5, 30:20:48.5:1.5, 25:25:48.5:1.5, 60:5:33:2, 50:10:38:2, 40:15:43:2, 30:20:48:2, 25:25:48:2, 60:5:32.5:2.5, 50:10:37.5:2.5, 40:15:42.5:2.5, 30:20:47.5:2.5, 25:25:47.5:2.5, 60:5:32:3, 50:10:37:3, 40:15:42:3, 30:20:47:3, and 25:25:47:3.

In addition, the composition may further comprise a prophylactic or therapeutic agent, and the agent may be a gene comprising RNA, DNA, or a mixture thereof in single-stranded or doublestranded form, and specifically may be at least one selected from the group consisting of small interfering RNA (siRNA), ribosomal RNA (rRNA), RNA, DNA, complementary DNA (cDNA), aptamer, messenger RNA (mRNA), transfer RNA (tRNA), antisense oligonucleotide, short hairpin RNA (shRNA), microRNA (miRNA), asymmetric interfering RNA (aiRNA), Dicer-substrate RNA (dsRNA), ribozyme, peptide nucleic acid (PNA), deoxyribozyme (DNAzyme), and single guide RNA (sgRNA) for gene editing, but is not limited thereto.

In addition, the composition may include various anionic peptides, protein drugs, protein-nucleic acid conjugates or anionic biopolymer-drug conjugates such as hyaluronic acid-peptide conjugates, hyaluronic acid-protein conjugates, and antibodies as active ingredients, but is not limited thereto.

The lipid nanoparticle composition including ionizable lipids, neutral lipids, steroids, polymerized lipids, and the like according to the present invention may be dissolved in ethanol or a solvent miscible with water to be prepared as a lipid solution. Separately, the active ingredient may be dissolved in citric acid or acetic acid buffer at pH 4.0±1.0 to prepare an active ingredient solution. In addition, the lipid solution and the active ingredient solution may be mixed at a volume ratio of 1:3 at a flow rate of about 10-15 mL/min using a microfluidic mixing device (Benchtop NanoAssemblr, Precision Nanosystems) to prepare lipid nanoparticles.

In addition, the present invention provides a drug delivery composition comprising: a lipid nanoparticle composition comprising the compound represented by Chemical Formula 1, a pharmaceutically acceptable salt thereof, a tautomer or a stereoisomer; and a prophylactic or therapeutic agent.

The prophylactic or therapeutic agent may be at least one selected from the group consisting of small interfering RNA (siRNA), ribosomal RNA (rRNA), RNA, DNA, complementary DNA (cDNA), aptamer, messenger RNA (mRNA), transfer RNA (tRNA), antisense oligonucleotide, short hairpin RNA (shRNA), microRNA (miRNA), asymmetric interfering RNA (aiRNA), Dicer-substrate RNA (dsRNA), ribozyme, peptide nucleic acid (PNA), deoxyribozyme (DNAzyme), single guide RNA (sgRNA) for gene editing, and mixtures thereof, but is not limited thereto.

The drug delivery composition may be administered to mammals including humans through various routes including parenteral administration, and the parenteral administration may be applied intravenously, subcutaneously, intraperitoneally, or topically, and the dosage may vary depending on the condition and weight of the patient, the degree of disease, the drug form, the administration route and time, but may be appropriately selected by those skilled in the art.

When the drug delivery composition according to an example is formulated, it is prepared using diluents or excipients such as commonly used fillers, extenders, lyophilized desiccants, binders, wetting agents, disintegrants, surfactants, and the like.

Formulations for parenteral administration include sterilized aqueous solutions, non-aqueous solutions, suspension, emulsions, lyophilized formulations, suppositories, etc.

As the non-aqueous solutions and the suspension agent, propylene glycol, polyethylene glycol, vegetable oil such as olive oil, injectable esters such as ethyl oleate, and the like may be used. As the base of the suppository, witepsol, macrogol, tween 61, cacao butter, laurin butter, glycerol, gelatin, and the like may be used.

The drug delivery composition of the present invention may be administered while including a pharmaceutically effective amount of a prophylactic or therapeutic agent. The effective dose level of the prophylactic or therapeutic agent may be determined according to the type of disease of the patient, the severity of the disease, the activity of the drug, the sensitivity to the drug, the time of administration, the route of administration and the excretion ratio, the treatment period, factors including the combination drug, and other factors well known in the medical field. The composition according to an embodiment may be administered as an individual therapeutic agent or in combination with another therapeutic agent, may be administered sequentially or simultaneously with a conventional therapeutic agent, and may be administered singly or multiply. In consideration of all of the above factors, it is important to administer an amount capable of obtaining the maximum effect in a minimum amount without side effects, which may be easily determined by those skilled in the art. For example, the composition may be administered at 0.01 to 100 mg/kg, 0.1 to 50 mg/kg, or 1 to 10 mg/kg.

### Modes for Carrying Out the Invention

Hereinafter, examples will be described in detail to help understanding of the present invention. However, the following examples are merely illustrative of the contents of the present invention, and the scope of the present invention is not limited to the following examples. Embodiments of the present invention are provided to more completely describe the present invention to those skilled in the art.

### <Example 1> Preparation of Compound

### 1-1. Preparation of Compound 1

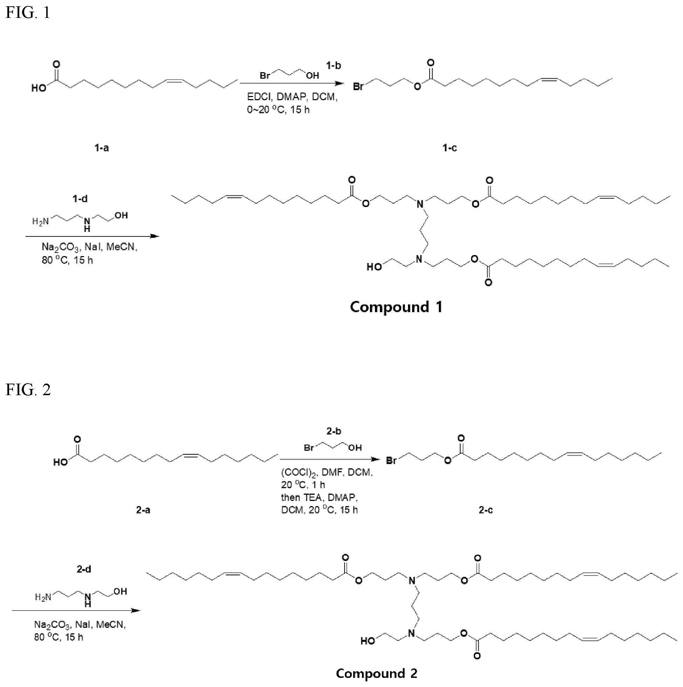

As shown in FIG. 1, Compound 1-a (3.00 g, 13.2 mmol), Compound 1-b (2.76 g, 19.9 mmol), and dimethylaminopyridine (DMAP; 810 mg, 6.63 mmol) were dissolved in dichloromethane (DCM; 60 mL), 1-ethyl-3-(3-dimethylaminopropyl)carbodiimide (EDCI; 5.59 g 29.2 mmol) was added at 0°C, and the mixture was stirred at 20°C for 15 hours. The mixture was diluted with purified water (100 mL), extracted with DCM (80 mL×3 times), washed with saturated sodium chloride solution (brine; 100 mL), dried with sodium sulfate (hereinafter, referred to as Na₂SO₄), filtered, and concentrated under reduced pressure, and purified on silica gel (petroleum ether (PE)/ethyl acetate (EtOAc)=100/1 to 19/1) to obtain compound 1-c of colorless oil.

Compound 1-c (2.00 g, 5.76 mmol), Compound 1-d (206 mg, 1.74 mmol), sodium carbonate (Na₂CO₃; 555 mg, 5.23 mmol), and sodium iodide (NaI; 262 mg, 1.74 mmol) were added to acetonitrile (MeCN; 25 mL) and stirred at 80°C for 15 hours. The mixture was concentrated under reduced pressure, diluted with purified water (40 mL), and the extracted solution with DCM (40 mL×3 times) was washed with brine (60 mL). After drying with Na₂SO₄, the filtered solution was concentrated under reduced pressure, purified twice on silica gel (PE/EtOAc=100/1 to 24/1), and lyophilized to obtain Compound 1 of colorless oil, and ¹H NMR analysis of Compound 1 showed as follows.
¹H NMR (400 MHz, CDCl₃) δ 5.31-5.39 (m, 6H), 4.08-4.13 (m, 6H), 3.57-3.58 (m, 2H), 2.42-2.59 (m, 12H), 2.29 (t, *J* = 7.6 Hz, 6H), 2.00-2.03 (m, 12H), 1.73-1.81 (m, 8H), 1.60-1.64 (m, 6H), 1.31-1.35 (m, 36H), 0.89-0.92 (m, 9H)

### 1-2. Preparation of Compound 2

As shown in FIG. 2, Compound 2-a (3.00 g, 11.8 mmol) was dissolved in DCM (30 mL), dimethylformamide (DMF; 86.2 mg, 1.18 mmol) and oxalyl chloride (COCl)₂; 3.34 g, 26.3 mmol) were added thereto at 0°C, and the mixture was stirred at 20°C for 1 hour. The mixture was concentrated under reduced pressure, diluted with DCM (20 mL), and then DCM (20 mL) in which Compound 2-b (2.13 g, 15.3 mmol, 1.39 mL), DMAP (144 mg, 1.18 mmol), and triethylamine (TEA; 5.97 g, 59.0 mmol, 8.21 mL) was dissolved was added thereto at 0°C, and the mixture was stirred at 20°C for 15 hours. The mixture was concentrated under reduced pressure, diluted with purified water (50 mL), and the extracted solution with EtOAc (50 mL×2 times) was washed with brine (60 mL). After drying with Na₂SO₄, the filtered solution was concentrated under reduced pressure and purified on silica gel (PE/EtOAc=100/1 to 20/1) to obtain Compound 2-c of colorless oil.

Compound 2-c (2.00 g, 5.33 mmol), Compound 2-d (197 mg, 1.66 mmol), Na₂CO₃ (529 mg, 4.99 mmol), and NaI (249 mg, 1.66 mmol) were added to MeCN (25 mL), and the mixture was stirred at 80°C for 15 hours. The mixture was diluted with purified water (30 mL) and the extracted solution with EtOAc (30 mL×3 times) was washed with brine (40 mL). After drying with Na₂SO₄, the filtered solution was concentrated under reduced pressure and purified on silica gel (DCM/ Methaol (MeOH)=100/1 to 24/1) to obtain Compound 2 as a colorless oil, and ¹H NMR analysis of Compound 2 showed as follows.

¹H NMR (500 MHz, CD₃OD) δ 5.32-5.38 (m, 6H), 4.11-4.15 (m, 6H), 3.62 (t, *J* = 6.0 Hz, 2H), 2.51-2.64 (m, 12H), 2.32 (t, *J* = 7.0 Hz, 6H), 2.00-2.05 (m, 12H), 1.76-1.85 (m, 6H), 1.60-1.66 (m, 8H), 1.30-1.33 (m, 48H), 0.89-0.92 (m, 9H).

### 1-3. Preparation of Compound 3

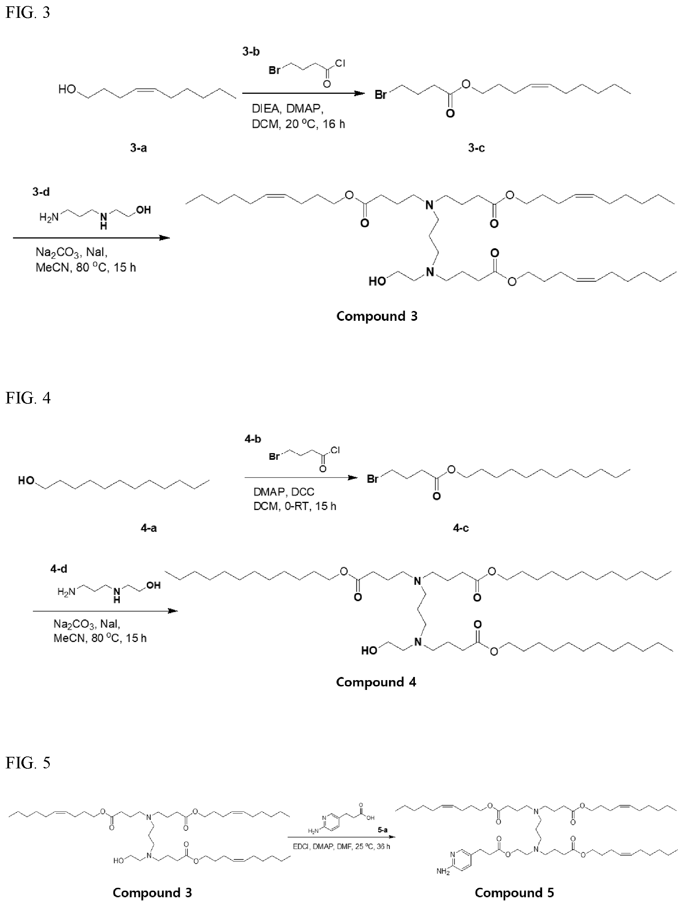

As shown in FIG. 3, Compound 3-a (5.00 g, 32.0 mmol), DMAP (301 mg, 2.46 mmol), and diisopropylethylamine (DIEA; 9.54 g, 73.8 mmol) were dissolved in DCM (50 mL), and then DCM (50 mL) in which Compound 3-b (4.56 g, 24.6 mmol, 2.85 mL) was dissolved was added thereto at 0°C, and the mixture was stirred at 20°C for 16 hours. The mixture was diluted with distilled water (100 mL), adjusted to pH 3 using a hydrochloric acid solution (2 M), extracted with DCM (100 mL × 2 times), washed with saturated aqueous sodium bicarbonate solution (sat. aq. NaHCO₃; 200 mL × 2 times), dried over sodium sulfate (Na₂SO₄), and filtered. The filtrate was concentrated under reduced pressure and then purified on silica gel (PE/EtOAc=100/1 to 20/1) to obtain Compound 3-c of colorless oil.

Compound 3-c (2.00 g, 6.55 mmol), Compound 3-d (235 mg, 1.99 mmol), Na₂CO₃ (631 mg, 5.96 mmol), and NaI (297.6 mg, 1.99 mmol) were added to MeCN (25 mL), and the mixture was stirred at 80°C for 15 hours. The mixture was diluted with purified water (30 mL) and the extracted solution with EtOAc (30mL×2 times) was washed with brine (40 mL). After drying with Na₂SO₄, the filtered solution was concentrated under reduced pressure and purified on silica gel (DCM/MeOH=100/1 to 24/1) to obtain Compound 3 of colorless oil, and ¹H NMR analysis of Compound 3 showed as follows.

¹H NMR (400 MHz, CD₃OD) δ 5.34-5.44 (m, 6H), 4.07 (t, *J* = 6.4 Hz, 6H), 3.63 (t, *J* = 6.0 Hz, 2H), 2.66 (t, *J* = 6.0 Hz, 2H), 2.50-2.63 (m, 10H), 2.37-2.39 (m, 6H), 2.10-2.14 (m, 6H), 2.04-2.06 (m, 6H), 1.76-1.83 (m, 6H), 1.64-1.73 (m, 8H), 1.31-1.35 (m, 18H), 0.89-0.93 (m, 9H).

### 1-4. Preparation of Compound 4

As shown in FIG. 4, Compound 4-a (200 mg, 1.0 mmol), DMAP (65 mg, 0.53 mmol), and N,N'-dicyclohexylcarbodimide (DCC; 65 mg, 0.53 mmol) were dissolved in DCM (20 mL), Compound 4-b (270 mg, 1.6 mmol) was added at 0°C, and the mixture was stirred in 25°C (RT) for 15 hours. After the mixture was diluted with purified water (20 mL), the solution extracted with DCM (20 mL×2 times) was washed with brine (40 mL), dried with Na₂SO₄, and the filtered solution was concentrated under reduced pressure, and then purified on silica gel (n-hexane/EtOAc=100/1 to 24/1) to obtain Compound 4-c of colorless oil.

Compound 4-c (121 mg, 0.363 mmol), Compound 4-d (13 mg, 0.11 mmol), Na₂CO₃ (35 mg, 0.33 mmol), and NaI (16.2 mg, 0.11 mmol) were added to MeCN (2.5 mL), and the mixture was stirred at 80°C for 15 hours. The mixture was diluted with purified water (10 mL) and the extracted solution with EtOAc (30 mL×3 times) was washed with brine (50 mL). After drying with Na₂SO₄, the filtered solution was concentrated under reduced pressure and purified on silica gel (DCM/MeOH=100/1 to 19/1) to obtain Compound 4 of colorless oil, and ¹H NMR analysis of Compound 4 showed as follows.

¹H NMR (400 MHz, Methanol-*d*₄) δ 4.05(t,*J* = 6.6 Hz, 6H), 3.59 (t, *J* = 6.2 Hz, 2H), 2.60 (t, *J* = 6.2 Hz, 2H), 2.52 (dd, *J* = 8.4, 6.2 Hz, 4H), 2.49 - 2.42 (m, 6H), 2.33 (td, *J* = 7.1, 3.9 Hz, 6H), 1.75 (h, *J* = 7.5 Hz, 6H), 1.67 - 1.54 (m, 8H), 1.41 - 1.19 (m, 56H), 0.92 - 0.83 (m, 9H).

### 1-5. Preparation of Compound 5

As shown in FIG. 5, Compound 5-a (315 mg, 1.90 mmol), Compound 3 (1.00 g, 1.26 mmol), and DMAP (386 mg, 3.16 mmol) were dissolved in DMF (10 mL), EDCI (363 mg, 1.90 mmol) was added thereto, and the mixture was stirred at 25°C for 12 hours. Compound 5-a (315 mg, 1.90 mmol) was added with DMAP (386 mg, 3.16 mmol), EDCI (363 mg, 1.90 mmol), and DMF (10 mL), and the mixture was stirred at 25°C for 24 hours. A saturated sodium carbonate solution (sat. Na₂CO₃, 30 mL) was added to the reaction mixture, and the resulting mixture was extracted with DCM (50 mL × 2 times). The combined organic layers were washed with brine (50 mL × 2 times). After drying with Na₂SO₄, the filtered solution was concentrated under reduced pressure and purified on silica gel (DCM/MeOH=100/1 to 100/3) to obtain Compound 5 of yellow oil, and ¹H NMR analysis of Compound 5 showed as follows.

¹H NMR (400 MHz, CDCl₃) δ 7.92(d,*J* = 2.0 Hz, 1H), 7.30 (dd, *J* = 8.4, 2.4 Hz, 1H), 6.46 (d, *J* = 8.4 Hz, 1H), 5.27 - 5.48 (m, 6H), 4.34 (s, 2H), 4.02-4.14 (m, 8H), 2.77-2.85 (m, 2H), 2.65 (t, *J* = 6.4 Hz, 2H), 2.52-2.60 (m, 2H), 2.36-2.50 (m, 10H), 2.28-2.36 (m, 6H), 2.04-2.12 (m, 6H), 1.95-2.02 (m, 6H), 1.65-1.79 (m, 12H), 1.48-1.57 (m, 2H), 1.26-1.39 (m, 18H), 0.89 (t, *J* = 6.8 Hz, 9H).

### 1-6. Preparation of Compound 6

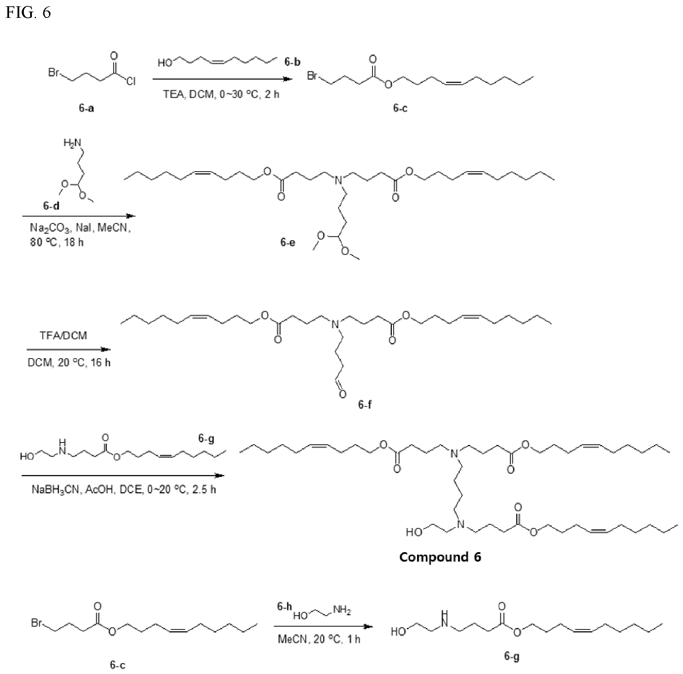

As shown in FIG. 6, Compound 6-b (5.54 g, 35.5 mmol) and TEA (10.8 g, 106 mmol) were dissolved in DCM (60 mL) at 0°C, and DCM (80 mL) in which Compound 6-a (8.55 g, 46.1 mmol) was dissolved was added thereto and stirred at 30°C for 2 hours. After the mixture was diluted with purified water (100 mL), the solution extracted with DCM (100 mL×3 times) was washed with brine (200 mL). The mixture was dried with Na₂SO₄, filtered and concentrated under reduced pressure, and then purified on silica gel (PE/EtOAc = 100/1 to 100/3) to obtain Compound 6-c of colorless oil. Compound 6-c (3.70 g, 12.1 mmol), Compound 6-d (734 mg, 5.51 mmol), Na₂CO₃ (1.75 mg, 16.5 mmol), and NaI (826 mg, 5.51 mmol) were added to MeCN (50 mL), and the mixture was stirred at 80°C for 18 hours. The mixture was diluted with distilled water (100 mL), extracted with EtOAc (100 mL × 3 times), and the combined organic layers were washed with aq. Na₂CO₃ (100 mL) and brine (100 mL). After drying with Na₂SO₄, the filtered solution was concentrated under reduced pressure and purified on silica gel (DCM/MeOH=100/1 to 25/1) to obtain Compound 6-e of light yellow oil.

Compound 6-e (1.30 g, 2.23 mmol) was dissolved in DCM (15 mL), and then trifluoroacetic acid (TFA; 1.5 mL) was added thereto, and the mixture was stirred at 20°C for 16 hours. The mixture was concentrated under reduced pressure, diluted with DCM (40 mL), washed with sat. aq. NaHCO₃ (40 mL × 3 times), dried with Na₂SO₄, filtered, and then concentrated under reduced pressure to obtain Compound 6-f as yellow oil.

Compound 6-c (1.44 g, 4.72 mmol) and Compound 6-h (2.88 g, 47.2 mmol) were added to MeCN (20 mL) and stirred at 20°C for 1 hour. The mixture was diluted with distilled water (50 mL), extracted with DCM (40 mL × 3 times), and the combined organic layers were washed with aq. Na₂CO₃ (50 mL), dried with Na₂SO₄, filtered, and the filtrate was concentrated under reduced pressure to obtain Compound 6-g.

Compound 6-f (1.15 g, 2.15 mmol), Compound 6-g (1.32 g, 3.24 mmol), and acetic acid (AcOH, 64.5 mg, 1.07 mmol) were added to dichloroethane (DCE; 20 mL), and the mixture was stirred at 20°C for 0.5 hour. Sodium cyanoborohydride (NaBH₃CN, 270 mg, 4.29 mmol) was added thereto at 0°C and stirred at 20°C for 2 hours. The mixture was diluted with distilled water (40 mL), extracted with dichloromethane (DCM, 30 mL × 3 times), and the combined organic layers were washed with aq. Na₂CO₃ (40 mL) and brine (40 mL). After drying with Na₂SO₄, the filtered solution was concentrated under reduced pressure, purified twice on silica gel (DCM/MeOH=100/1 to 100/7), purified with prep-HPLC (C4 150 mm*19 mm; purified water:ACN=100/10 to 100/80), and lyophilized. The dried product was diluted with DCM (30 mL), washed with aq. Na₂CO₃ (30 mL) and brine (30 mL), dried with Na₂SO₄, filtered, concentrated under reduced pressure, and purified by silica gel column chromatography (DCM/MeOH = 100/1 to 100/6) to obtain Compound 6 as a colorless oil, and the ¹H NMR analysis of Compound 6 showed the following results.

¹H NMR (400 MHz, CDCl₃) δ 5.26-5.50(m,6H),4.00-4.15(m,6H),3.48-3.67 (m,2H),2.40-2.71(m,12H),2.34(t,*J* = 7.2 Hz, 6H), 2.09-2.13 (m, 6H), 2.00-2.04 (m, 6H), 1.74-1.89 (m, 6H), 1.65-1.73 (m, 6H), 1.40-1.58 (m, 4H), 1.25-1.39 (m, 18H), 0.80-0.96 (m, 9H).

### 1-7. Preparation of Compound 7

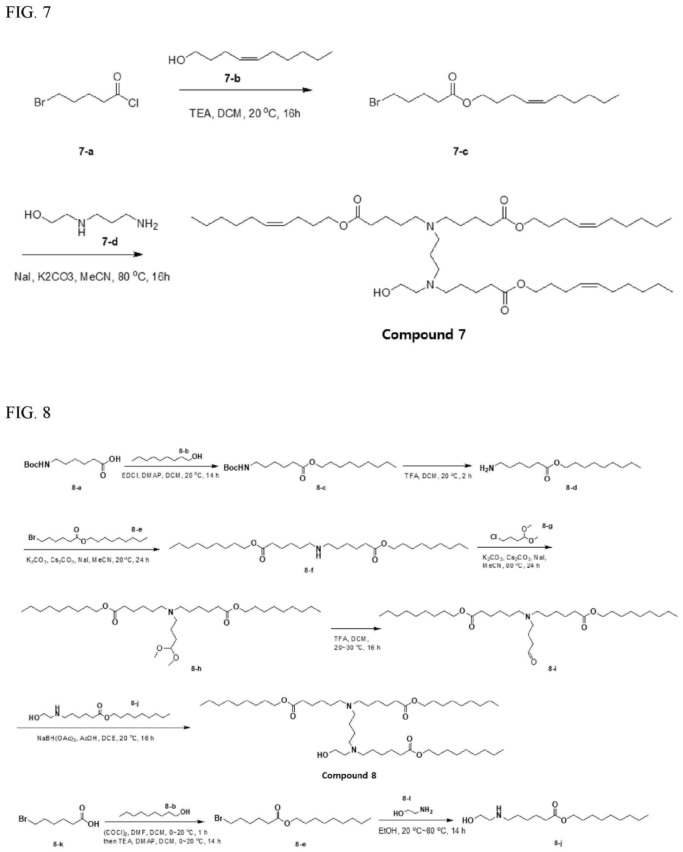

As shown in FIG. 7, Compound 7-b (5.00 g, 32.0 mmol) and TEA (9.71 g, 96.0 mmol) were dissolved in DCM (50 mL), and then DCM (70 mL) in which Compound 7-a (7.02 g, 35.2 mmol) was dissolved was added at 0°C and stirred at 20°C for 16 hours. After the mixture was diluted with purified water (30 mL), the solution extracted with EtOAc (30 mL×2 times) was washed with brine (20 mL), dried with Na₂SO₄, and the filtered solution was concentrated under reduced pressure, and then purified on silica gel (PE/EtOAc=100/1 to 100/6) to obtain Compound 7-c of colorless oil.

Compound 7-c (3.00 g, 9.40 mmol), Compound 7-d (336 mg, 2.85 mmol), K₂CO₃ (1.06 mg, 9.97 mmol), and NaI (427 mg, 2.85 mmol) were added to MeCN (30 mL), and the mixture was filled with nitrogen gas and stirred at 80°C for 16 hours. The mixture was diluted with distilled water (50 mL), extracted with EtOAc (50 mL × 2 times), and the combined organic layers were washed with aq. Na₂CO₃ (30 mL) and brine (30 mL). After drying with Na₂SO₄, the filtered solution was concentrated under reduced pressure, purified twice on silica gel (DCM/MeOH=100/1 to 100/4), purified with prep-HPLC (C4 150 mm*19 mm; purified water:MeCN=100/10 to 100/80), and lyophilized. The dried product was diluted with DCM (30 mL), washed with aq. Na₂CO₃ (30 mL), dried with Na₂SO₄, filtered, and concentrated under reduced pressure to afford Compound 7 as a yellow oil, and the ¹H NMR analysis of Compound 7 showed the following results.

¹H NMR (400 MHz, CDCl₃) δ 5.29-5.46(m,6H),4.07(t,*J* = 6.8 Hz, 6H), 3.47 - 3.63 (m, 2H), 2.37-2.60 (m, 12H), 2.33 (t, *J* = 7.6 Hz, 6H), 2.08-2.14 (m, 6H), 1.99-2.04 (m, 6H), 1.58-1.73 (m, 14H), 1.45-1.53 (m, 6H), 1.26-1.37 (m, 18H), 0.90 (t, *J* = 7.2 Hz, 9H).

### 1-8. Preparation of Compound 8

As shown in FIG. 8, Compound 8-a (25.0 g, 108 mmol) and Compound 8-b (17.2 g, 119 mmol) were dissolved in DCM (300 mL), and a mixed solution in which DMAP (13.5 g, 110 mmol) and EDCI (27.1 g, 142 mmol) were dissolved in DCM (300 mL) was added thereto, and the mixture was stirred at 20°C for 14 hours. The mixture was diluted with purified water (300 mL), extracted with DCM (400 mL×2 times), washed with brine (300 mL), dried with Na₂SO₄, and filtered filtrate was concentrated under reduced pressure, and then purified in silica gel (PE/EtOAc=100/1 to 12/1) to obtain Compound 8-c of colorless oil.

Compound 8-c (34.7 g, 97.1 mmol) was dissolved in DCM (175 mL), TFA (175 mL) was added thereto, and the mixture was stirred at 20°C for 2 hours. The mixture was concentrated under reduced pressure and then diluted with DCM (200 mL) and purified water (200 mL), adjusted to pH 10 with 1M NaOH aqueous solution, and extracted with DCM (200 mL×3 times). The solution was washed with 0.1M NaOH aqueous solution (250 mL×2 times) and brine (300 mL), dried with Na₂SO₄, and filtered, and concentrated under reduced pressure to obtain Compound 8-d of colorless oil.

Compound 8-k (17.6 g, 90.1 mmol) was dissolved in DCM (200 mL), DMF (507 mg, 6.93 mmol) and oxalyl chloride ((COCl)₂, 26.4 g, 208 mmol) were added at 0°C, and the mixture was stirred at 20°C for 1 hour, concentrated under reduced pressure, and diluted with DCM (100 mL). DCM (150 mL) in which compound 8-b (10.0 g, 69.3 mmol), DMAP (847 mg, 6.93 mmol), and TEA (35.1 g, 347 mmol) was dissolved was added at 0°C thereto and the mixture was stirred at 20°C for 14 hours. The reaction mixture was diluted with distilled water (200 mL), adjusted to pH 3 using a 2 M hydrochloric acid solution, extracted with DCM (200 mL × 2), and the combined organic layers were washed with sat. NaHCO₃ (300 mL × 2) and brine (300 mL). After drying with Na₂SO₄, the filtered solution was concentrated under reduced pressure and purified on silica gel (PE/EtOAc=100/1 to 16/1) to obtain Compound 8-e of colorless oil.

Compound 8-d (9.59 g, 37.3 mmol), Compound 8-e (3.99 g, 12.4 mmol), potassium carbonate (K₂CO₃, 5.15g, 37.3 mmol), cesium carbonate (Cs₂CO₃, 1.21g, 3.73 mmol), and NaI (558 mg, 3.73 mmol) were added to MeCN (200 mL), and the mixture was stirred at 20°C for 24 hours. The mixture was filtered, and the filtration residue was washed with DCM (200 mL), and the filtrate was filtered under reduced pressure and then purified on silica gel (DCM/MeOH=100/1 to 32/1) to obtain compound 8-f of a light yellow solid.

Compound 8-f (2.34 g, 4.70 mmol) and Compound 8-g (2.15 g, 14.1 mmol) were dissolved in MeCN (50 mL), potassium carbonate (K₂CO₃, 1.30 g, 9.40 mmol), cesium carbonate (Cs₂CO₃, 1.53 g, 4.70 mmol), and NaI (705 mg, 4.70 mmol) were added thereto, and the mixture was stirred at 80°C for 24 hours. The mixture was concentrated to remove MeCN, diluted with DCM (50 mL) and purified water (50 mL), extracted with DCM (50 mL×3 times), washed with brine (80 mL), dried with Na₂SO₄, filtered, and concentrated under reduced pressure, and purified on silica gel (DCM/MeOH=100/1 to 32/1) to obtain compound 8-h of pale yellow oil.

Compound 8-h was dissolved in DCM (18 mL), TFA (1.80 mL) was added thereto, and the mixture was stirred at 20°C. for 14 hours, stirred at 30°C for 2 hours, and then concentrated under reduced pressure. The mixture was diluted with DCM (50 mL), washed with aq. Na₂CO₃ (50 mL × 3), dried with Na₂SO₄, filtered, and concentrated under reduced pressure to obtain Compound 8-i of a yellow oil.

Compound 8-e (3.00 g, 9.34 mmol) was dissolved in ethanol (EtOH, 80 mL), Compound 8-l (8.56 g, 14.0 mmol) was added at 20°C, and the mixture was stirred at 80°C for 14 hours. The mixture was concentrated, diluted with distilled water (200 mL), extracted with EtOAc (30 mL), and the combined organic layers were washed with sat. NaHCO₃ (30 mL) and brine (40 mL), dried with Na₂SO₄, filtered, and concentrated under reduced pressure to obtain Compound 8-j of a white solid.

Compound 8-i (800 mg, 1.41 mmol), Compound 8-j (425 mg, 1.41 mmol), and acetic acid (AcOH, 42.3 mg, 0.704 mmol) were added to DCE (10 mL), and the mixture was stirred at 20°C for 1 hour. Sodium-Triacetosyborohydride (NaBH(OAc)₃; 597 mg, 2.82 mmol) was added thereto and stirred at 20°C for 15 hours. The mixture was diluted with purified water (30 mL), extracted with DCM (30 mL×3 times), and washed with brine (50 mL). The solution dried with Na₂SO₄ and filtered was concentrated under reduced pressure and purified on silica gel (DCM/MeOH=100/1 to 10/1) to obtain Compound 8 of a colorless oil, and ¹H NMR analysis of Compound 8 showed as follows.

¹H NMR (400 MHz, CD₃OD) δ 4.07 (t, *J* = 6.8 Hz, 6H), 3.77-3.80 (m, 2H), 2.99-3.03 (m, 12H), 2.34-2.39 (m, 6H), 1.59-1.73 (m, 22H), 1.31-1.45 (m, 42H), 0.89-0.92 (m, 9H).

### 1-9. Preparation of Compound 9

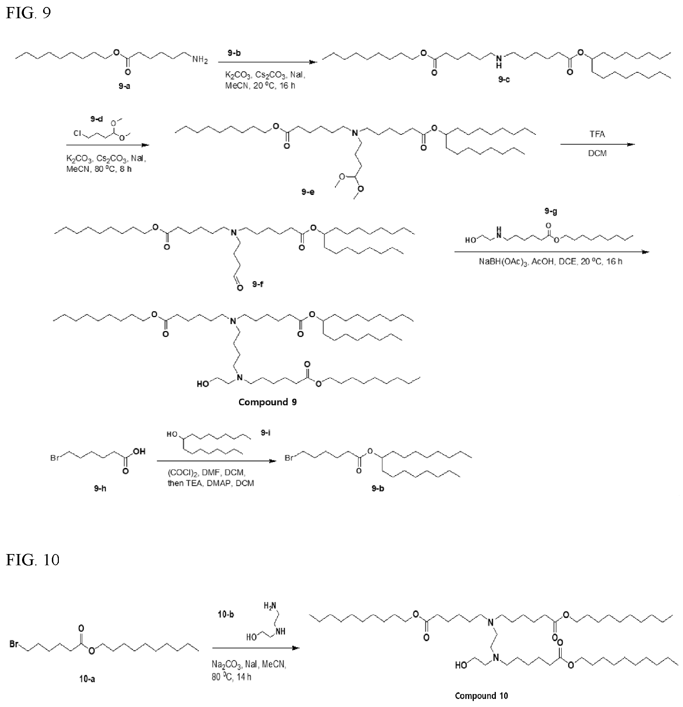

As shown in FIG. 9, compound 9-h (7.42 g, 38.0 mmol) was dissolved in DCM (200 mL), DMF (214 mg, 2.92 mmol) and oxalyl chloride ((COCl)₂, 11.1g, 87.7 mmol) were added thereto at 0°C, and the mixture was stirred at 20°C for 1 hour. The mixture was concentrated under reduced pressure, diluted with DCM (100 mL). DCM (120 mL) in which Compound 9-i (7.50 g, 29.2 mmol, 1.39 mL), DMAP (357 mg, 2.92 mmol), and TEA (14.8 g, 146 mmol) was dissolved was added at 0°C thereto and the mixture was stirred at 20°C for 14 hours. The mixture was diluted with distilled water (200 mL), adjusted to pH 3 using a 2 M hydrochloric acid solution, extracted with DCM (200 mL × 2), and the combined organic layers were washed with sat. NaHCO₃ (300 mL × 2). After drying with Na₂SO₄, the filtered solution was concentrated under reduced pressure and purified on silica gel (PE/EtOAc=100/1 to 16/1) to obtain Compound 9-b of colorless oil.

Compound 9-a (9.59 g, 37.3 mmol) was added to MeCN (200 mL), and Compound 9-b (5.38 g, 12.4 mmol), potassium carbonate (K₂CO₃, 5.15 g, 37.3 mmol), cesium carbonate (Cs₂CO₃, 1.21 g, 3.73 mmol), and NaI (558 mg, 3.73 mmol) were added thereto, and the mixture was stirred at 20°C for 16 hours. The mixture was filtered and the filtrate was filtered under reduced pressure and then purified twice on silica gel (DCM/MeOH=100/1 to 32/1) to obtain compound 9-c of colorless oil.

Compound 9-c (600 mg, 984 umol) and Compound 9-d (450 mg, 2.95 mmol) were added to MeCN (10 mL), potassium carbonate (K₂CO₃, 408 mg, 2.95 mmol), cesium carbonate (Cs₂CO₃, 96.1 mg, 0.295 mmol), and NaI (147 mg, 0.984 mmol) were added thereto, and the mixture was stirred at 80°C for 8 hours. The mixture was concentrated, diluted with purified water (40 mL), extracted with EtOAc (40 mL), washed with brine, dried with Na₂SO₄, and filtered, concentrated under reduced pressure, and purified on silica gel (DCM/MeOH=100/1 to 32/1) to obtain compound 9-e of colorless oil.

Compound 9-e (200 mg, 0.275 mol) was dissolved in DCM (2 mL), and then TFA (307 mg, 2.69 mmol) was added thereto at 20°C, and the mixture was stirred at 20°C for 28 hours. The mixture was concentrated under reduced pressure, washed with sat. aq. NaHCO₃ (20 mL), extracted with EtOAc (20 mL), washed with brine, dried with Na₂SO₄, filtered, and concentrated under reduced pressure to obtain Compound 9-f as a colorless oil.

Compound 9-f (150 mg, 0.221 mmol) and compound 9-g (79.8 mg, 0.265 mmol) were dissolved in DCE (3 mL), and then acetic acid (42.3 mg, 0.704 mmol) was added dropwise thereto at 20°C, and the mixture was stirred at 20°C for 1 hour. NaBH(OAc)₃ (93.5 mg, 0.441 mmol) was added thereto, and the mixture was stirred at 20°C for 15 hours. The mixture was diluted with purified water (20 mL), extracted with DCM (20 mL×3 times), and washed with brine (30 mL). The solution dried with Na₂SO₄ and filtered was concentrated under reduced pressure and purified on silica gel (DCM/MeOH=100/1 to 19/1) to obtain compound 9 of light yellow oil, and ¹H NMR analysis of compound 9 showed as follows.

¹H NMR (400 MHz, CDCl₃)δ4.83-4.88(m,1H),4.06(t,*J* = 6.8 Hz, 4H), 3.70-3.71 (m, 2H), 2.69-2.79 (m, 12H), 2.30-2.32 (m, 6H), 1.60-1.71 (m, 20H), 1.50-1.52 (m, 4H), 1.26-1.39 (m, 54H), 0.87-0.90 (m, 12H).

### 1-10. Preparation of Compound 10

As shown in FIG. 10, Compound 10-a (2.99 g, 8.91 mmol), Compound 10-b (290 mg, 2.78 mmol), Na₂CO₃ (885 mg, 8.35 mmol), and NaI (417 mg, 2.78 mmol) were added to MeCN (30 mL), and the mixture was stirred at 80°C for 14 hours. The mixture was diluted with purified water (30 mL) and the solution extracted with EtOAc (30 mL×3 times) was washed with brine (50 mL). After drying with Na₂SO₄, the filtered solution was concentrated under reduced pressure and then purified twice on silica gel (DCM/MeOH=100/1 to 20/1) to obtain Compound 10 of light yellow oil, and ¹H NMR analysis of Compound 10 showed as follows.

¹H NMR (500 MHz, CD₃OD)δ 4.05-4.08 (m, 6H), 3.61 (t, *J* = 5.5 Hz, 2H), 2.83-2.86 (m, 2H), 2.69-2.75 (m, 4H), 2.67 (t, *J* = 5.5 Hz, 2H), 2.57-2.62 (m, 2H), 2.32-2.36 (m, 6H), 1.49-1.66 (m, 18H), 1.30-1.37 (m, 50H), 0.89-0.92 (m, 9H).

### 1-11. Preparation of Compound 11

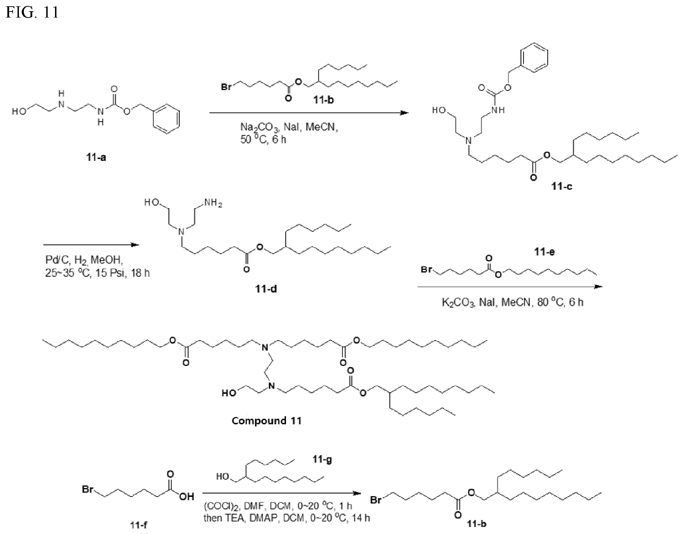

As shown in FIG. 11, Compound 11-f (10.5 g, 53.6 mmol) was dissolved in DCM (100 mL), DMF (301 mg, 4.12 mmol) and oxalyl chloride (15.7 g, 124 mmol) were added at 0°C, and then stirred at 20°C for 1 hour. The mixture was concentrated under reduced pressure, diluted with DCM (80 mL). DCM (120 mL) in which 11-g (10.0 g, 41.2 mmol), DMAP (504 mg, 4.12 mmol), and TEA (20.9 g, 206 mmol) was dissolved was added at 0°C thereto and the mixture was stirred at 20°C for 14 hours. The mixture was diluted with distilled water (100 mL), adjusted to pH 3 using a 2 M hydrochloric acid solution, extracted with DCM (150 mL × 2), and the combined organic layers were washed with sat. NaHCO₃ (200 mL) and brine (150 mL). After drying with Na₂SO₄, the filtered solution was concentrated under reduced pressure and purified on silica gel (PE/EtOAc=100/1 to 16/1) to obtain compound 11-b of colorless oil.

Compound 11-a (14.0 g, 29.4 mmol), Compound 11-b (4.11 g, 9.79 mmol), Na₂CO₃ (2.08 g, 19.6 mmol), and NaI (1.47 g, 9.79 mmol) were added to MeCN (210 mL), and the mixture was stirred at 50°C for 6 hours (2 batches). Two batch preparations were filtered and concentrated under reduced pressure and then purified on silica gel (DCM/MeOH=100/1 to 24/1) to obtain Compound 11-c of yellow oil.

Compound 11-c (790 mg, 1.37 mmol) was added to MeOH (10 mL), and palladium/carbon catalyst (Pd/C, 80 mg, purity 10%) was added under nitrogen gas, and then hydrogen (H₂) was replaced, and the mixture was stirred at 25°C for 4 hours under hydrogen gas (15 Psi), and then the mixture was stirred at 35°C for 14 hours. The reaction was filtered, the residue was washed with MeOH (20 mL) after filtration, concentrated under reduced pressure, and purified on silica gel (0.2% NH₃.MeOH in DCM/MeOH=100/1 to 3/1) to obtain Compound 11-d of yellow oil.

Compound 11-d (300 mg, 0.678 mmol), Compound 11-e (682 mg, 2.03 mmol), K₂CO₃ (281 mg, 2.03 mmol), and NaI (50.8 mg, 0.339 mmol) were added to MeCN (13 mL), and the mixture was stirred at 80°C for 6 hours. The mixture was diluted with purified water (50 mL) and the extracted solution with DCM (50 mL×2 times) was washed with brine (50 mL). After drying with Na₂SO₄, the filtered solution was concentrated under reduced pressure, purified on silica gel (DCM/MeOH=100/1 to 19/1), and lyophilized to obtain Compound 11 of colorless oil, and ¹H NMR analysis of Compound 11 showed as follows.

¹H NMR (400 MHz, CDCl₃) δ 4.06 (t, *J* = 6.8 Hz, 4H), 3.97 (d, *J* = 6.0 Hz, 2H), 3.60-3.61 (m, 2H), 2.47-2.68 (m, 12H), 2.31 (t, *J* = 7.2 Hz, 6H), 1.59-1.69 (m, 13H), 1.51-1.52 (m, 4H), 1.27-1.31 (m, 58H), 0.87-0.90 (m, 12H).

### 1-12. Preparation of Compound 12

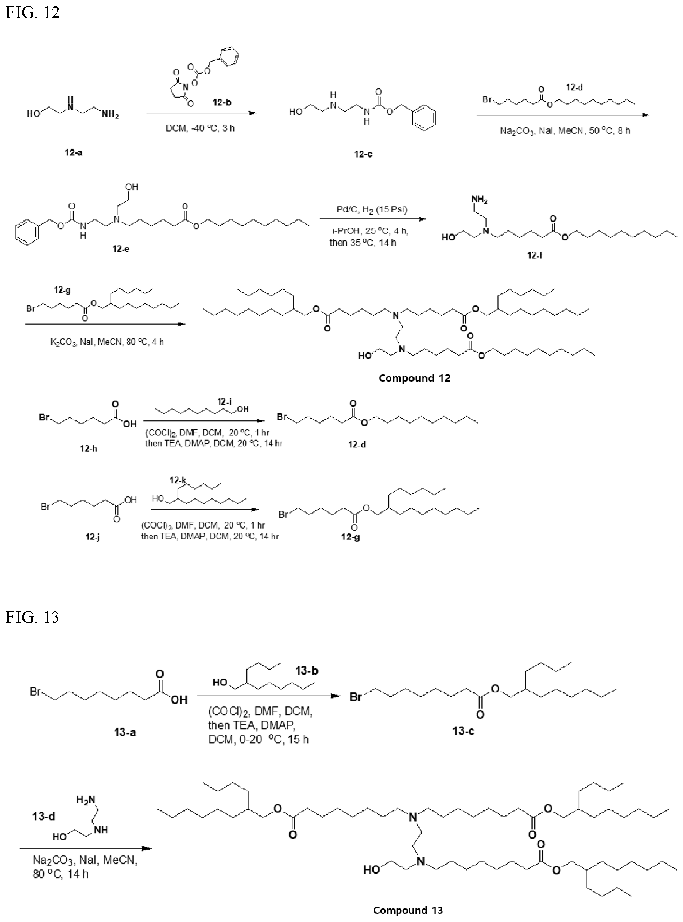

As shown in FIG. 12, Compound 12-b (23.9 g, 96 mmol) was dissolved in DCM (800 mL), and Compound 12-a (10.0 g, 96.0 mmol) dissolved in DCM (800 mL) was added under -40°C and nitrogen gas, stirred for 3 hours, and concentrated under reduced pressure to obtain Compound 12-c of an off-white solid state.

Compound 12-c (20.0 g, 42.0 mmol), Compound 12-d (4.69 g, 14.0 mmol), Na₂CO₃ (2.97 g, 28.0 mmol), and NaI (2.10 g, 14.0 mmol) were added to MeCN (200 mL), and the mixture was stirred at 50°C for 8 hours. The mixture was filtered and concentrated under reduced pressure, purified by silica gel column chromatography (0.2% NH₃.MeOH in DCM/MeOH = 100/1 to 24/1), diluted with DCM (20 mL), and washed with sat. aq. NaHCO₃ (30 mL × 2). The solution dried over Na₂SO₄ and filtered was concentrated under reduced pressure to obtain Compound 12-e.

Compound 12-e (1.50 g, 3.04 mmol) was added to isopropyl alcohol (I-PrOH; 15 mL), a palladium/carbon catalyst (Pd/C, 150 mg, purity 10%) was added thereto under nitrogen gas, and then hydrogen was replaced, and the mixture was stirred at 25°C for 4 hours under hydrogen gas (15 Psi), and then the mixture was stirred at 35°C for 14 hours. The reaction was filtered, the residue was washed with isopropyl alcohol (20 mL), concentrated under reduced pressure, and purified on silica gel (0.2% NH₃.MeOH in DCM/MeOH=100/1 to 1/100) to obtain compound 12-f of colorless oil.

Compound 12-h (16.0 g, 82.1 mmol) was dissolved in DCM (200 mL), DMF (462 mg, 6.32 mmol) and oxalyl chloride ((COCl)₂, 24.1 g, 190 mmol) were added at 0°C, and the mixture was stirred at 20°C for 1 hour. The mixture was concentrated under reduced pressure, diluted with DCM (150 mL), and then, a mixed solution in which Compounds 12-i (10.0 g, 63.2 mmol), DMAP (772 mg, 6.32 mmol), and TEA (32.0 g, 316 mmol) were dissolved in DCM (100 mL) at 0°C was added thereto, and the mixture was stirred at 20°C for 14 hours. After completion of the reaction, the mixture was diluted with distilled water (200 mL), adjusted to pH 3 using a 2 M hydrochloric acid solution, extracted with DCM (200 mL × 2), and the combined organic layers were washed with sat. NaHCO₃ (300 mL × 2). After drying with Na₂SO₄, the filtered solution was concentrated under reduced pressure and purified on silica gel (PE/EtOAc=100/1 to 24/1) to obtain compound 12-d of colorless oil.

Compound 12-j (10.5 g, 53.6 mmol) was dissolved in DCM (200 mL), DMF (301 mg, 4.12 mmol) and oxalyl chloride ((COCl)₂, 15.7 g, 124 mmol) were added at 0°C, and the mixture was stirred at 20°C for 1 hour. The mixture was concentrated under reduced pressure, diluted with DCM (100 mL). DCM (100 mL) in which compound 12-k (10.0 g, 41.3 mmol), DMAP (504 mg, 4.12 mmol), and TEA (20.9 g, 206 mmol) was dissolved was added at 0°C thereto and the mixture was stirred at 20°C for 14 hours. After completion of the reaction, the mixture was diluted with purified water (200 mL), adjusted to pH 3 using a 2 M hydrochloric acid solution, extracted with DCM (200 mL × 2), and the organic layer was washed with sat. NaHCO₃ (300 mL × 2). After drying with Na₂SO₄, the filtered solution was concentrated under reduced pressure and purified on silica gel (PE/EtOAc=100/1 to 19/1) to obtain compound 12-g of colorless oil.

Compound 12-f (250 g, 0.697 mmol), Compound 12-g (877 mg, 2.09 mmol), K₂CO₃ (289 mg, 2.09 mmol), and NaI (52.3mg, 0.349 mmol) were added to MeCN (10 mL), and the mixture was stirred at 80°C for 4 hours. The reaction was concentrated under reduced pressure, diluted with purified water (30 mL), and the solution extracted with EtOAc (30 mL×2 times) was washed with brine (30 mL). The solution dried with Na₂SO₄ and filtered was concentrated under reduced pressure and then purified on silica gel (DCM/MeOH=100/1 to 19/1) to obtain Compound 12 of colorless oil, and ¹H NMR analysis of Compound 12 showed as follows.

¹H NMR (400 MHz, CD₃OD) δ 4.07 (t, *J* = 6.8 Hz, 2H), 4.00 (d, *J* = 5.6 Hz, 4H), 3.47-3.72 (m, 2H), 2.41-3.20 (m, 12H), 2.27-2.37 (m, 6H), 1.63-1.68 (m, 10H), 1.48-1.55 (m, 6H), 1.28-1.37 (m, 68H), 0.89-0.92 (m, 15H).

### 1-13. Preparation of Compound 13

As shown in FIG. 13, Compound 13-a (15.6 g, 69.8 mmol) was dissolved in DCM (100 mL), DMF (392 mg, 5.37 mmol) and oxalyl chloride ((COCl)₂, 20.4 g, 161 mmol) were added at 0°C, and then stirred at 20°C for 1 hour. The mixture was concentrated under reduced pressure, diluted with DCM (80 mL). DCM (100 mL) in which compound 13-b (10.0 g, 54 mmol), DMAP (656 mg, 5.37 mmol), and TEA (27.2 g, 268 mmol) was dissolved was added at 0°C thereto and the mixture was stirred at 20°C for 14 hours. After completion of the reaction, the mixture was diluted with distilled water (100 mL), adjusted to pH 3 using a 2 M hydrochloric acid solution, extracted with DCM (150 mL × 2), and the combined organic layers were washed with sat. NaHCO₃ (150 mL × 2) and brine (150 mL). After drying with Na₂SO₄, the filtered solution was concentrated under reduced pressure and purified on silica gel (PE/EtOAc=100/1 to 50/1) to obtain compound 13-c of colorless oil.

Compound 13-c (6.01 g, 15.4 mmol), compound 13-d (500 mg, 4.80 mmol), Na₂CO₃ (1.53 g, 14.4 mmol), and NaI (719 g, 4.80 mmol) were added to MeCN (50 mL), and the mixture was stirred at 80°C for 14 hours. The solution was diluted with purified water (30 mL) at room temperature and the solution extracted with EtOAc (30 mL×3 times) was washed with brine (50 mL). The solution dried with Na₂SO₄ and filtered was concentrated under reduced pressure and purified on silica gel (DCM/MeOH=100/1 to 20/1) to obtain Compound 13 of yellow oil, and ¹H NMR analysis of Compound 13 showed as follows.

¹H NMR (400 MHz, CD₃OD) δ 4.02 (d, *J* = 5.6 Hz, 6H), 3.65 (t, *J*=5.2 Hz, 2 H), 2.79-3.14 (m, 8H), 2.63-2.76 (m, 4H), 2.30-2.39 (m, 6H), 1.62-1.70 (m, 12H), 1.50-1.60 (m, 3H), 1.30-1.43 (m, 66H), 0.88-1.00 (m, 18H).

### <Example 2> Preparation of Lipid Nanoparticles

Lipid nanoparticles in which Firefly Luciferase mRNA (RNAgene, Korea) was encapsulated were prepared using the compound (ionizable lipid) prepared in Example 1, dioleoylphosphatidylethanolamine (DOPE), or distearoylphosphatidylcholine (DSPC) (neutral lipid), cholesterol (steroids), and PEG 2000-DMG (polymerized lipid). However, in the case of Compound 7, lipid nanoparticles in which Firefly Luciferase saRNA (Microuni, Korea) was encapsulated instead of Firefly Luciferase mRNA were prepared. A lipid solution was prepared by dissolving the ionizable lipid, DOPE/DSPC, cholesterol, and PEG 2000-DMG in ethanol at a concentration of 4.8mg/mL in a mole percent ratio of 40:15:43.5:1.5, and an active ingredient solution was prepared by separately dissolving Firefly Luciferase mRNA in a citric acid buffer of pH 4.0±1.0 or an acetic acid buffer of pH 5.0±1.0 so that the mRNA: ionizable lipid was in a weight ratio of 1:10. Thereafter, lipid nanoparticles were prepared by passing the lipid solution and the active ingredient solution through a microfluidic mixing device (Benchtop Nanoassemblr, Precision Nanosystems) at a flow rate of about 15 mL/min so as to have a volume ratio of 1:3. The prepared lipid nanoparticles were diluted with Tris buffer containing 8.7% sucrose and dialyzed with a dialysis centrifuge tube to have an ethanol content of less than 1%, and the final concentration was prepared to be 0.2mg/mL based on mRNA.

For comparison, lipid nanoparticles in which Firefly Luciferase mRNA (RNA, Korea) was encapsulated were prepared using ALC-0315 ionizable lipid, DSPC (neutral lipid), cholesterol (steroid), and ALC-0159 (polymerized lipid). ALC-0315, DSPC, cholesterol, and ALC-0159 were prepared in the same way at a molar % ratio of 46.3:9.4:42.7:1.6 and used as ALC-0315 lipid nanoparticles for comparison.

### <Example 3> Evaluation of Lipid Nanoparticle Size and Polydispersity

The size and surface charge of the lipid nanoparticles according to the ionizable lipid prepared in Example 1 were measured. In order to measure the size of lipid nanoparticles, the lipid nanoparticles were diluted using PBS such that the concentration of mRNA contained in each lipid nanoparticle was 1 µg/mL, and the diameter and polydispersity (polydispersity index; PDI) of the lipid nanoparticles were measured using Dynamic Light Scattering (DLS) in Mlvern Zetasizer Nano (Malvern Instruments, UK).

As a result, as shown in Table 1, all of the lipid nanoparticles showed a size of about 150 nm or less and a good polydispersity within 0.2.

**[Table 1]**

| Compound No. | particle size(nm) | PDI |
|---|---|---|
| 1 | 108 | 0.07 |
| 2 | 113 | 0.07 |
| 3 | 99 | 0.05 |
| 4 | 102 | 0.13 |
| 5 | 122 | 0.03 |
| 6 | 104 | 0.06 |
| 7 | 89 | 0.06 |
| 8 | 98 | 0.11 |
| 9 | 118 | 0.1 |
| 10 | 106 | 0.11 |
| 11 | 98 | 0.05 |
| 12 | 104 | 0.1 |

### <Example 4> Evaluation of Gene Encapsulation Efficiency

In order to confirm the encapsulation efficiency of the lipid nanoparticles, Quant-iT^{™} RiboGreen RNA Reagent and Kit was used. A ribosomal RNA standard (100 µg/mL in TE buffer) was taken in 5 µL and diluted with 245 µL TE (Tris-EDTA) buffer or 0.4% Triton-TE buffer, respectively, to prepare a stock solution for calibration. The stock solution (2, 5, 10, 25, and 50 µL) was taken and diluted with TE buffer or 0.4% Triton-TE buffer to adjust the total volume to 100 µL, and then 100 µL of Quant-iT^{™} RiboGreen RNA Reagent was added and mixed. The fluorescence intensity (excitation 475 nm and Emission 500~550 nm) was measured to prepare a calibration curve. Separately, 5 µL of the lipid nanoparticle solution prepared in Example 2 was taken and diluted with 245 µL of TE buffer (10 mM Tris-HCl, 1 mM EDTA, pH 7.5 in DEPC-treated water). Then, 50 µL of diluted lipid nanoparticle solution was mixed with 50 µL of TE buffer or 2% Triton-TE buffer, respectively, and incubated at 37°C for approximately 10 min. Thereafter, 40 µL of each mixture was transferred to a microplate, followed by the sequential addition of 60 µL TE buffer and 100 µL of Quant-iT^{™} RiboGreen RNA Reagent. The fluorescence intensity was measured and substituted into the calibration curve to calculate the gene amount (T0) in TE buffer and the gene amount (T2) in 2% Triton-TE buffer. The gene encapsulation efficiency was calculated using Equation 1 below. $Gene encapsulation efficiency \left(%\right) = \frac{\left(T2-T0\right)}{T 2} ∗ 100$

As a result, as shown in Table 2, it was confirmed that all of the compounds showed good encapsulation efficiencies of 85% or more.

**[Table 2]**

| Compound No. | Gene encapsulation efficiency(%) |
|---|---|
| 1 | 88.7 |
| 2 | 89.2 |
| 3 | 86.1 |
| 4 | 95.4 |
| 5 | 71.2 |
| 6 | 84.4 |
| 7 | 93.0 |
| 8 | 95.9 |
| 9 | 95.5 |
| 10 | 91.6 |
| 11 | 90.3 |
| 12 | 92.1 |

### <Example 5> Evaluation of Gene Delivery Efficiency of Lipid Nanoparticle

### 5-1. Evaluation of Efficiency of Intracellular Gene Delivery

In order to confirm the intracellular gene delivery efficiency of the lipid nanoparticles, lipid nanoparticles (FLuc mRNA-LNP) were used. In order to evaluate the gene delivery efficiency, HeLa cells (Korean Cell Line Bank, Seoul), which are human cervical cancer cell lines expressing LDL receptors on the cell surface, were used. The cells were cultured in MEM medium supplemented with 10% fetal bovine serum and penicillin/streptomycin at 37°C in a 5% CO₂ incubator. The HeLa cells were seeded into a 96-well plate at 2×10⁴ cells per well and incubated. After 16 to 24 h, the lipid nanoparticles were diluted in MEM medium containing 10% FBS at a concentration of 0.25 µg/ml on the basis of encapsulated mRNA, and 100 µl per well was added to the cells for treatment.

In order to confirm the expression of luciferase in the cells delivered through the lipid nanoparticles, the culture medium in each well was removed at 6 hours after treatment, and the cells were washed with PBS. Then, 100 µl of Glo-Lysis Buffer (Promega, WI, USA) was added to lyse the cells at room temperature for 5 minutes. To the lysed cells, 100 µl of Steady-Glo^{™} Luciferase Assay solution (Promega) was added at a 1:1 ratio and incubated at room temperature for 5 minutes. The mixture was then transferred to a 96-well white plate, and luminescence was measured using a GloMax Discover microplate reader (Promega). A calibration curve was generated using recombinant luciferase (Promega) as a standard, and the luciferase concentration (pg/ml) of the samples was calculated therefrom.

As a result, as shown in Table 3, it was confirmed that the lipid nanoparticles comprising the ionizable lipid of the present invention showed high gene delivery efficiency *in vitro*. From the above results, it was confirmed that the lipid nanoparticles may be utilized as gene carriers.

**[Table 3]**

| Compound No. | Luciferase expression level(mean±SD, pg/ml) |
|---|---|
| 3 | 228,260±18,472 |
| 7 | 338,847±4,760 |
| 9 | 15,811±3,398 |
| 10 | 3,545±217 |
| 12 | 67,540±1,204 |

### 5-2. Evaluation of Gene Delivery Efficiency in vivo

To confirm the *in vivo* gene delivery efficiency and distribution of lipid nanoparticles, the lipid nanoparticles were administered either by a single intravenous injection at a dose of 1 mg/kg or by intramuscular injection at a dose of 0.5 mg/kg per site into both femoral regions, based on the encapsulated gene. However, in the case of lipid nanoparticles (containing saRNA) of Compound 7, intramuscular injections were performed at a dose of 0.05 mg/kg into both femoral regions. In the case of intravenous injection, mice were sacrificed at 4 hours after administration, and the liver was collected. In the case of intramuscular injection, organs including femoral muscles were collected either 4 hours or 6 hours after administration.

In order to confirm the luciferase expression level in the tissue samples, the weight of each tissue section was measured. In the case of liver tissue, the tissue was transferred to a tube containing 3 mm metal beads, and 500 µl of Glo-Lysis Buffer (Promega) per 50 mg of tissue was added, followed by homogenization using a bead homogenizer. In the case of femoral muscle, 500 µl of Glo-Lysis Buffer (Promega) per 100 mg of tissue was added, followed by homogenization using a rotor/stator homogenizer. The supernatant of the centrifuged lysate was taken and diluted with Glo-Lysis Buffer at an appropriate ratio, and 50 µl of the diluted tissue lysate and 50 µl of Steady-Glo Luciferase Assay solution were mixed in a 1:1 ratio. After incubation for 5 minutes, the luminescence value of luciferase was measured in the same manner as in Example 3-1, and the expression amount of luciferase (ng/g tissue) was calculated.

As a result, as shown in Table 4, it was confirmed that the lipid nanoparticles comprising the ionizable lipid of the present invention showed optimal levels of protein expression in liver tissues following intravenous injection, thereby showing optimal gene delivery efficiency following systemic administration.

**[Table 4]**

| Compound No. | Luciferase expression levels in the liver after intravenous injection (Mean ± SD, ng/g tissue) |
|---|---|
| 3 | 12,066±3,744 |
| 9 | 2,658±881 |
| 10 | 1,311±424 |

In addition, as shown in Table 5, it was confirmed that the lipid nanoparticles comprising the ionizable lipid of the present invention exhibited local gene delivery efficiency in muscle tissue and minimized diffusion into liver tissue following intramuscular injection. Compared with the conventional ionizable lipids, which have a problem in that a large amount of ionizable lipid leaks from the administration site even upon intramuscular injection and thus causes a high level of gene expression, particularly in the liver, it was confirmed that the lipid nanoparticles of the present invention exhibit excellent gene delivery efficiency in the administrated muscle tissue upon intramuscular injection.

**[Table 5]**

| Compound No. | Time after administration | Luciferase expression levels in the muscle and liver after intramuscular injection (Mean ± SD, ng/g tissue) | |
|---|---|---|---|
| | | Muscle (combined value from both thighs) | Liver |
| 4 a) | 4 | 437±136 | 0.3±0.08 |
| 5 ^{a)} | 6 | 2,078±318 | 31±15 |
| 6 a) | 6 | 2,321±677 | 384±346 |
| 7 b) | 6 | 397±240 | 1.8±1.0 |

| | | | |
|---|---|---|---|
| a) 0.5 mg/kg dose, b) 0.05 mg/kg dose | | | |

In addition, as shown in Table 6, it was confirmed that the lipid nanoparticles according to Compound 3 of the present invention showed high muscle delivery upon intramuscular injection, and the ratio of the expression level in the liver to that in the muscle was less than 5%, indicating that the total systemic exposure was very low. On the other hand, lipid nanoparticles comprising ALC-0315 ionizable lipid, which was used as a comparator, leaked in large amount from the administration site, and the ratio of the expression in the liver to that in the muscle exceeded 100%, confirming that systemic exposure was very high. Considering most lipid nanoparticles are delivered to the liver during systemic exposure due to the characteristics of lipid nanoparticles, it was confirmed that the ionizable lipid of the present invention has the characteristic of primarily expressing genes in muscle tissue upon intramuscular injection.

**[Table 6]**

| Compound No. | Time after administration | Luciferase expression levels in the muscle and liver after local administration (Mean ± SD, ng/g tissue) | |
|---|---|---|---|
| | | Muscle (combined value from both thighs) | Liver |
| 3 | 6 | 5,764±1,989 | 118±87 |
| ALC-0315 | 6 | 2,264±1,092 | 2,748±1,979 |

For reference, it is known that lipid nanoparticle-based mRNA vaccines, after intramuscular administration, (1) primarily deliver genes to muscle cells, (2) secondarily deliver genes to dendritic cells and macrophages in muscle tissue, and (3) ultimately induce completion of the immune response involving B cells and T cells within lymph nodes of the muscle tissue. Since immune responses of the mRNA vaccine are completed locally in muscle tissue through this process, it is important to minimize unnecessary systemic exposure of lipid nanoparticles, which may cause excessive systemic immunity, in order to reduce systemic side effects of the vaccine and to express optimal effects. In this respect, it was confirmed that the ionizable lipid of the present invention not only encapsulates mRNA into lipid nanoparticles to effectively deliver the same, but also minimizes systemic exposure through selective muscle delivery, thereby providing useful clinical advantages in the development of mRNA vaccines for infectious diseases and cancer, as well as in application for the treatment of genetic muscle diseases.

As described above in detail with respect to specific aspects of the present invention, for those skilled in the art, it is clear that this specific description is only a preferred example, and the scope of the present invention is not limited thereby. Accordingly, the substantial scope of the present invention will be defined by the appended claims and their equivalents.

## Claims

1. A compound represented by Chemical Formula 1, or a pharmaceutically acceptable salt, tautomer, or stereoisomer thereof. 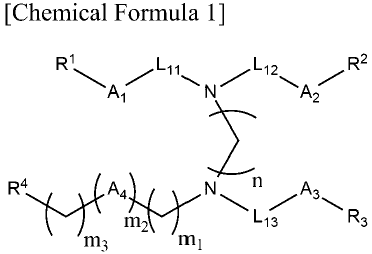 In Chemical Formula 1,
n is one selected from 1 to 6;
m₁ and m₃ are each independently one selected from 0 to 6;
m₂ is 0 or 1;
L₁₁, L₁₂, and L₁₃ are each the same or different, and selected from the group consisting of (C1-C10)alkylene, (C2-C10)alkenylene, and (C2-C10)alkynylene, wherein at least one -CH₂-groups in the alkylene, alkenylene, and alkynylene are optionally substituted with -O-, -S-, or - SS-;
A₁ to A₄ are each the same or different, and selected from the group consisting of -C(=O)-, - OC(=O)-, -C(=O)O-, -OC(=O)O-, -OP(=O)O-, -OC(=S)-, -C(=S)O-, -SC(=O)-, -C(=O)S-, - SC(=S)-, -C(=S)S-, -SC(=O)O-, -OC(=O)S-, -S-, -SS-, -C(=O)NH-, -NHC(=O)-, -C(=O)NR¹¹-, -NR¹¹C(=O)-, -OC(=O)NH-, -NHC(=O)O-, -OC(=O)NR¹¹-, and -NR¹¹C(=O)O-; R¹¹ is selected from the group consisting of (C1-C10)alkyl, (C2-C10)alkenyl, and (C2-C10)alkynyl, wherein at least one -CH₂- groups in the alkyl, alkenyl, and alkynyl are optionally substituted with -O-, -S-, or -SS-; and
R¹, R², R³, and R⁴ are each the same or different, and selected from the group consisting of hydroxy, (C1-C20)alkyl, (C2-C20)alkenyl, (C2-C20)alkynyl, (C3-C20)cycloalkyl, (C3-C20)aryl, and 4- to 20-membered heteroaryl comprising at least one heteroatom selected from the group consisting of N, O, and S, wherein at least one -CH₂- groups in the alkylene, alkenylene and alkynylene is optionally substituted with -O-, -S- or -SS-, and wherein the (C3-C20)cycloalkyl, (C3-C20)aryl, and 4- to 20-membered heteroaryl groups are each optionally substituted with at least one substituent selected from hydroxy, amino, halogen, and (C1-C5)alkyl.

2. The compound according to claim 1, wherein in Chemical Formula 1, n is one selected from 2 to 4; m₁ and m₃ are each independently selected from 0 to 4; L₁₁, L₁₂, and L₁₃ are each the same or different, and selected from the group consisting of (C3-C6)alkylene, (C3-C6)alkenylene, and (C3-C6)alkynylene; A₁ to A₄ are each the same or different, and selected from -OC(=O)- or -C(=O)O-; R¹, R², and R³ are each the same or different, and selected from the group consisting of (C5-C20)alkyl, (C5-C20)alkenyl, and (C5-C20)alkynyl, wherein at least one -CH₂- groups are optionally substituted with -O-, -S-, or -SS-; and R⁴ is hydroxy, (C3-C10)cycloalkyl, (C3-C10)aryl, or a 4- to 10-membered heteroaryl comprising at least one nitrogen atom, wherein the (C3-C10)cycloalkyl, (C3-C10)aryl, and 4- to 10-membered heteroaryl groups are each optionally substituted with amino.

3. The compound according to claim 1, wherein in Chemical Formula 1, n is one selected from 3 to 4; L₁₁, L₁₂, and L₁₃ are each independently (C3-C6)alkylene; R¹, R², and R³ are each the same or different, and selected from the group consisting of (C5-C20)alkyl, (C5-C20)alkenyl, and (C5-C20)alkynyl, wherein at least one -CH₂- groups are optionally substituted with -S- or -SS-; and R⁴ is hydroxy or a 4- to 10-membered heteroaryl comprising one nitrogen atom, wherein the heteroaryl group is optionally substituted with amino.

4. The compound according to claim 1, wherein the compound is selected from the group consisting of compounds:
(1) (9Z,9'Z)-((3-((2-hydroxyethyl)(3-((Z)-tetradec-9-enoyloxy)propyl)amino) propyl)azanediyl)bis(propane-3,1-diyl)bis(tetradec-9-enoate);
(2) (9Z,9'Z)-((3-((3-((Z)-hexadec-9-enoyloxy)propyl)(2-hydroxyethyl)amino)propyl)azanediyl)bis(propane-3,1-diyl) bis(hexadec-9-enoate);
(3) di((Z)-dec-4-en-1-yl)4,4'-((3-((4-((Z)-dec-4-en-1-yloxy)-4-oxobutyl)(2-hydroxyethyl)amino)propyl)azanediyl)dibutanoate;
(4) didodecyl 4,4'-((3-((4-(dodecyloxy)-4-oxobutyl) (2-hydroxyethyl)amino)propyl)azanediyl)dibutanoate;
(5) di((Z)-dec-4-en-1-yl)4,4'-((3- ((2-((3-(6-aminopyridin-3-yl) propanoyl)oxy)ethyl)(4-((Z)-dec-4-en-1-yloxy)-4-oxobutyl)amino)propyl)azanediyl)dibutanoate;
(6) di((Z)-dec-4-en-1-yl)4,4'-((4-((4-((Z)-dec-4-en-1-yloxy)-4-oxobutyl) (2-hydroxyethyl)amino)butyl)azanediyl)dibutanoate;
(7) di((Z)-dec-4-en-1-yl)5,5'-((3-((5-((Z)-dec-4-en-1-yloxy)-5-oxopentyl) (2-hydroxyethyl)amino)propyl)azanediyl)dipentanoate;
(8) dinonyl6,6'-((4-((2-hydroxyethyl)(6-(nonyloxy) -6-oxohexyl)amino)butyl)azanediyl)dihexanoate;
(9) heptadecan-9-yl 6-((4-((2- hydroxyethyl)(6-(nonyloxy)-6-oxohexyl)amino)butyl)(6-(nonyloxy)-6-oxohexyl)amino)hexanoate;
(10) didecyl 6,6'-((2-((6-(decyloxy)-6-oxohexyl) (2-hydroxyethyl)amino)ethyl)azanediyl)dihexanoate;
(11) decyl 6-((2-((6-(decyloxy) -6-oxohexyl)(2-hydroxyethyl)amino)ethyl)(6-((2-hexyldecyl)oxy)-6-oxohexyl)amino)hexanoate;
(12) bis(2-hexyldecyl) 6,6'-((2-((6-(decyloxy) -6-oxohexyl)(2-hydroxyethyl)amino)ethyl)azanediyl)dihexanoate;
(13) di((Z)-dec-4-en-1-yl) 4,4'-((3-((4-((Z)-dec-4-en-1-yloxy)-4-oxobutyl)(pyridin-4-yl)amino)propyl)azanediyl)dibutanoate;
(14) di((Z)-tetradec-9-en-1-yl) 4,4'-((3-((2-hydroxyethyl)(4-oxo-4-((Z)-tetradec-9-en-1-yloxy)butyl)amino)propyl)azanediyl)dibutanoate;
(15) bis(6-(butyldisulfanyl)hexyl) 4,4'-((3-((4-((6-(butyldisulfanyl)hexyl)oxy)-4-oxobutyl)(3-hydroxypropyl)amino)propyl)azanediyl)dibutanoate;
(16) di((Z)-dec-4-en-1-yl)4,4'-((3-((2-((3-(4-aminophenyl)propanoyl)oxy) ethyl)(4-((Z)-dec-4-en-1-yloxy)-4-oxobutyl)amino)propyl)azanediyl)dibutanoate;
(17) di((Z)-dec-4-en-1-yl) 4,4'-((3-((4-aminophenethyl)(4-((Z)-dec-4-en-1-yloxy)-4-oxobutyl)amino)propyl)azanediyl)dibutanoate;
(18) di((Z)-dec-4-en-1-yl) 4,4'-((3-((4-((Z)-dec-4-en-1-yloxy)-4-oxobutyl)(4-hydroxybutyl)amino)propyl)azanediyl)dibutanoate; and
(19) (9Z,9'Z)-((3-((2-hydroxyethyl)(4-((Z)-tetradec-9-enoyloxy)butyl)amino)propyl)azanediyl)bis(butane-4,1-diyl) bis(tetradec-9-enoate).

5. A lipid nanoparticle composition comprising the compound of any one of claims 1 to 4.

6. The lipid nanoparticle composition according to claim 5, wherein the compound is an ionizable lipid.

7. The lipid nanoparticle composition according to claim 5, wherein the composition further comprises at least one additional lipid selected from the group consisting of neutral lipids, steroids, and polymerized lipids.

8. The lipid nanoparticle composition according to claim 7, wherein the neutral lipid is a phospholipid or glycolipid.

9. The lipid nanoparticle composition according to claim 7, wherein the steroids is at least one selected from the group consisting of cholesterol, bile acid derivatives, and cholic acid derivatives.

10. The lipid nanoparticle composition according to claim 7, wherein the polymerized lipid is a PEGylated lipid and is at least one selected from the group consisting of PEG-modified phosphatidylethanolamine, PEG-modified phosphatidic acid, PEG-modified ceramide, PEG-modified dialkylamine, PEG-modified diacylglycerol, and PEG-modified dialkylglycerol.

11. The lipid nanoparticle composition according to claim 7, wherein the composition comprises 20 to 65 mol% of ionizable lipid, 2.5 to 30 mol% of neutral lipid, 25 to 60 mol% of steroids

12. The lipid nanoparticle composition according to claim 5, wherein the composition further comprises a prophylactic or therapeutic agent.

13. The lipid nanoparticle composition according to claim 12, wherein the prophylactic or therapeutic agent is at least one selected from the group consisting of small interfering RNA (siRNA), ribosomal RNA (rRNA), RNA, DNA, complementary DNA (cDNA), aptamers, messenger RNA (mRNA), transfer RNA (tRNA), antisense oligonucleotides, short hairpin RNA (shRNA), microRNA (miRNA), asymmetric interfering RNA (aiRNA), Dicer-substrate RNA (dsRNA), ribozymes, peptide nucleic acids (PNA), deoxyribozymes (DNAzyme), a guide RNA for gene editing (sgRNA), and mixtures thereof.

14. A drug-delivery composition comprising: a lipid nanoparticle composition according to claim 5; and a prophylactic or therapeutic agent.

15. The drug-delivery composition according to claim 14, wherein the prophylactic or therapeutic agent is at least one selected from the group consisting of small interfering RNA (siRNA), ribosomal RNA (rRNA), RNA, DNA, complementary DNA (cDNA), aptamers, messenger RNA (mRNA), transfer RNA (tRNA), antisense oligonucleotides, short hairpin RNA (shRNA), microRNA (miRNA), asymmetric interfering RNA (aiRNA), Dicer-substrate RNA (dsRNA), ribozymes, peptide nucleic acids (PNA), deoxyribozymes (DNAzyme), guide RNA for gene editing (sgRNA), and mixtures thereof.
